(19) Europäisches Patentamt
European Patent Office
Office européen des brevets

(11) EP 4 389 756 A1

(12) # EUROPEAN PATENT APPLICATION
published in accordance with Art. 153(4) EPC

(43) Date of publication:
**26.06.2024 Bulletin 2024/26**

(21) Application number: **22857448.9**

(22) Date of filing: **02.07.2022**

(51) International Patent Classification (IPC):
***C07J 43/00*** *(2006.01)* ***A61K 31/58*** *(2006.01)*
***A61P 17/10*** *(2006.01)* ***A61P 35/00*** *(2006.01)*
***A61P 13/08*** *(2006.01)*

(86) International application number:
**PCT/CN2022/103497**

(87) International publication number:
**WO 2023/020135 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.08.2021 CN 202110948115**

(71) Applicant: **Guangdong Zhongke Medicine R&D Limited**
**Guangzhou, Guangdong 510663 (CN)**

(72) Inventors:
- **WANG, Wei**
  **Guagnzhou, Guangdong 510663 (CN)**
- **LU, Yongzhang**
  **Guagnzhou, Guangdong 510663 (CN)**
- **TAN, Jinhui**
  **Guagnzhou, Guangdong 510663 (CN)**

(74) Representative: **Isarpatent**
**Patent- und Rechtsanwälte**
**Barth Hassa Peckmann & Partner mbB**
**Friedrichstraße 31**
**80801 München (DE)**

(54) **ABIRATERONE DERIVATIVE AND PREPARATION AND APPLICATION THEREOF**

(57) Disclosed in the present invention are an abiraterone derivative and preparation method thereof. The abiraterone derivative provided by the present invention shows relatively few abdominal distension phenomena with respect to abiraterone and has better drug effect and tolerance, and pharmacokinetic experiments show that the plasma concentration of M1 after intragastric administration of equal molar dose is significantly higher than that of abiraterone acetate. The prostate cancer inhibiting effect is obviously better than that of abiraterone, and the side effects on the gastrointestinal tract is smaller than that of abiraterone. A compound provided by the present invention also has the effect of treating acne.

EP 4 389 756 A1

## Description

### TECHNICAL FIELD

The invention relates to the field of medicine, in paricular to abiraterone derivatives, preparation and application thereof.

### BACKGROUND

The structural formula of abiraterone is:

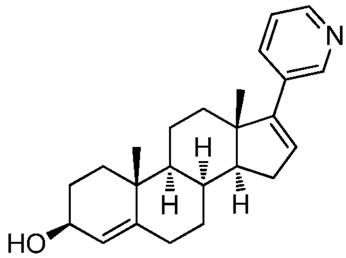

.

Abiraterone, which chemical name is 17-(3-pyridyl)androst-5,16-dien-3β-ol, is a CYP17 inhibitor. Abiraterone in combination with prednisone is mainly indicated for the treatment of patients with metastatic castration-resistant prostate cancer who have received prior chemotherapy containing docetaxel. Abiraterone acetate (ZYTIGA) is converted in vivo to abiraterone, an androgen biosynthesis inhibitor, that inhibits 17 α-hydroxylase/C17,20-lyase (CYP17). This enzyme is expressed in testicular, adrenal, and prostate tumor tissues and is required for androgen biosynthesis. Abiraterone is clinically used in the form of abiraterone acetate, which needs to be taken on an empty stomach due to the large influence of food on the absorption of abiraterone acetate, and there are also significant differences between individuals. One of the metabolites of abiraterone acetate, referred to as M1, which chemical name is: 17-(3-pyridine)-4, 16-dieneandrost-3-one, English name: 17-(3-pyridine)-4,16-dieneandrost-3-one, is one of the metabolites of Abiraterone acetate.

Previous research has shown that some tumor model mice inoculated with Vcap (human prostate cancer cells) have developed abdominal distension and died after receiving intraperitoneal injections of abiraterone acetate. Autopsy revealed that some of the drugs were not absorbed.

### SUMMARY OF THE INVENTION

The present invention provides an abiraterone derivative, preparation and application thereof. Compared with abiraterone, the abiraterone derivative provided by the present invention exhibits relatively less abdominal distension and has better drug efficacy and tolerance. The pharmacokinetic experiment shows that the plasma concentration of M1 (ZONK1901-1) was significantly higher than that of abiraterone acetate after intragastric administration of equimolar doses, and M2 (ZONK1901-2) was detected in the metabolites of M1 , while M2 was not detected in metabolites of abiraterone acetate. It shows that M1 has a significantly better inhibitory effect on prostate cancer than that of abiraterone, and has less gastrointestinal side effects than abiraterone. The compound provided by the present invention also has the effect of treating acne.

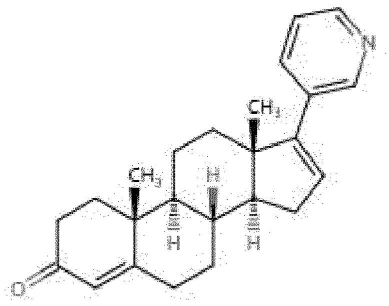

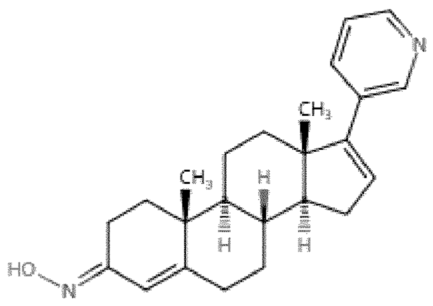

ZONK1901-1 ZONK1901-2

(M1) (M2)

Abiraterone derivatives provided by the present invention have a structural formula as shown in formula I:

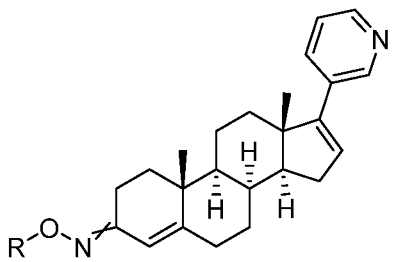

式I

In formula I, R is selected from:

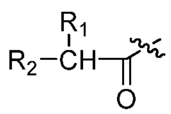 , 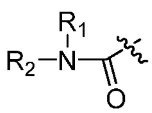 , 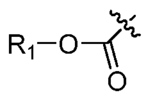 、 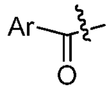

(Ar represents a substituted or unsubstituted aromatic ring, specifically a benzene ring or a pyridine ring; the substituent could be a C1-C8 alkyl group, a C1-C8 alkoxy group, or halogen) ;

Wherein, R1 or R2 is independently selected from any one of the following groups: H, (C3-C8) carbocycloalkyl, substituted or unsubstituted (C1-C18) (specifically, C2-C6) alkane (wherein, the substituent could be amino (amino at the terminal does not contain active hydrogen), carboxyl (carboxyl at the terminal does not contain active hydrogen), halogen, (C3-C8) heterocycloalkyl, (C2-C8) alkenes or (C2-C8) alkenyl containing substituents, (C2-C8) alkynyl or (C2-C8) alkynyl containing substituents, (C6-C20) aryl or (C6-C20 ) aryl containing substituents, (C2-C20) heterocyclic or (C2-C20) heterocyclic containing substituents;

Alternatively, R1 and R2 form a ring with each other as (C3-C8) heterocycloalkyl or substituted (C3-C8) heterocycloalkyl, (C6-C20) heteroaryl or substituted (C6-C20) heteroaryl;

When R is:

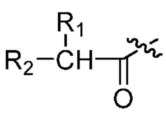

, R1 or R2 is not alkane with 1 or more unsubstituted carbon atoms;

The substituents of the compound do not include the terminal substituents being hydroxyl, amino, carboxyl, phosphoryl and sulfonyl, but include the compound in which the active hydrogen of hydroxyl, amino, carboxyl, phosphoryl and sulfonyl is substituted. It has been proved by experiments that the compounds with active hydrogen groups at the terminal are unstable. It is found in the experiment that, when R1 and R2 are unsubstituted amino groups or the terminals of R1 and R2 are unsubstituted amino groups, stable compound samples cannot be obtained. When the terminals of R1 and R2 are carboxyl group, stable compound samples also cannot be obtained.

When the terminal amino group is substituted or forms an amide bonded, the compounds shown below can be obtained and have the characteristics described in the present invention. When the terminal is a carboxyl group, the compound sample cannot be obtained for the reason of stability, but when the carboxyl group forms an ester with an alcohol or a substituted alcohol, or forms an amide with an amine or a substituted amine, and the terminal is not substituted by an amino group or a carboxyl group, stable samples can be obtained , and have the characteristics of the present invention.

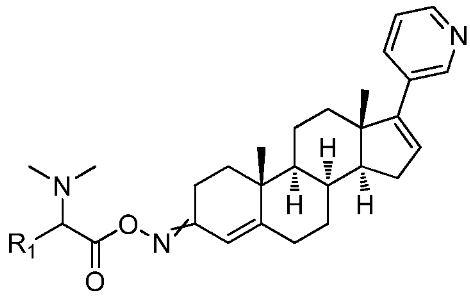
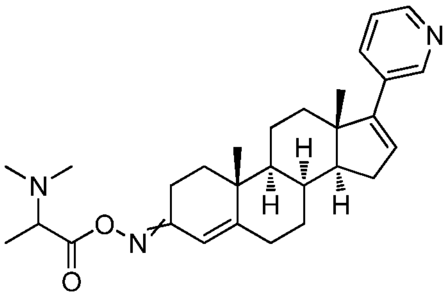

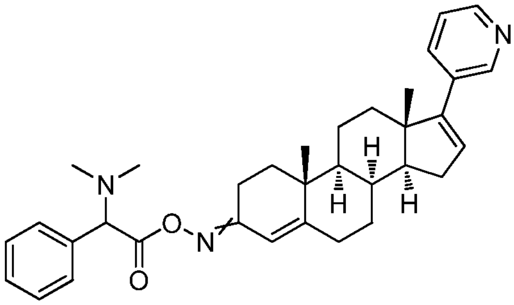
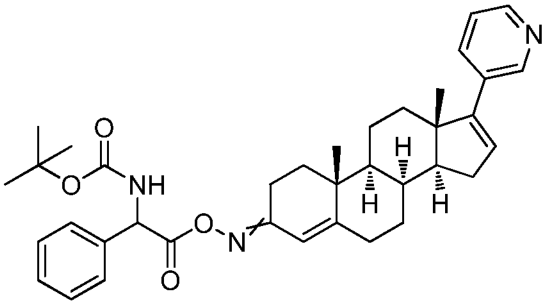

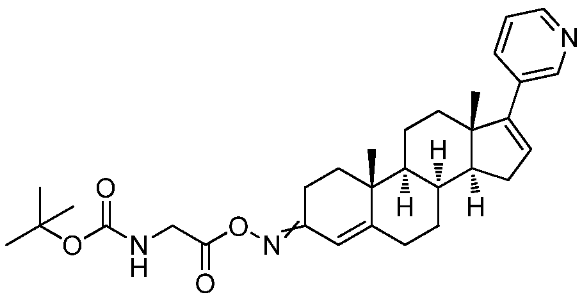
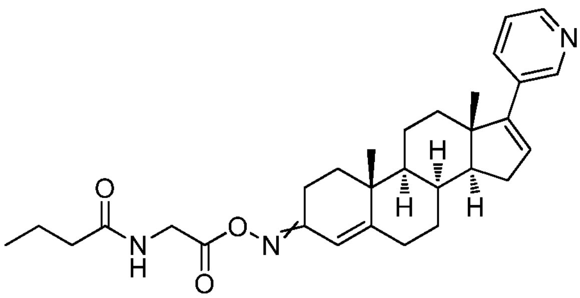

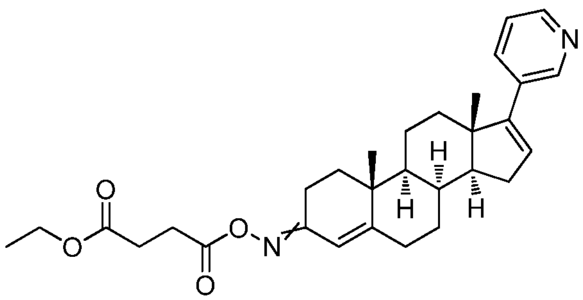

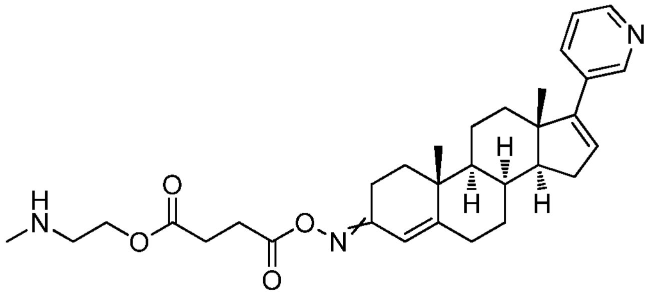

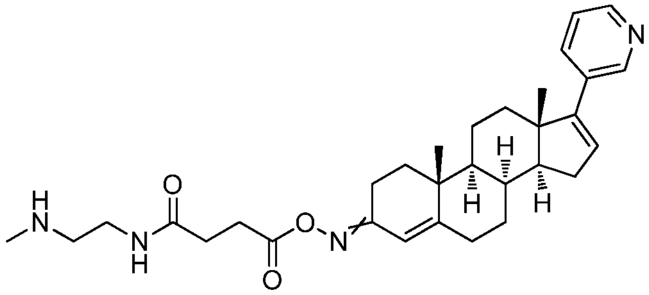

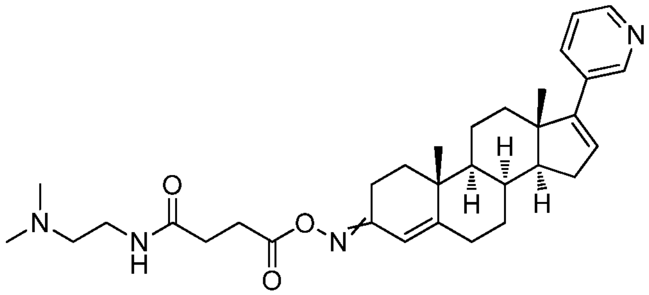

Specifically,

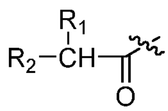

, R2 is H, and R1 is a straight-chain alkyl group with 1 or more carbon atoms. For example, when R1 is -CH3, the drug concentrations of M1 and M2 (see Pharmacokinetic Examples for structure) in the body are significantly lower than those of other compounds. It is because straight-chain alkyl groups, lipid solubility of which is greater, is difficult to absorb and hydrolyze in vivo.

Specifically, the representative compunds of the abiraterone derivative shown in the above formula I are as follows:

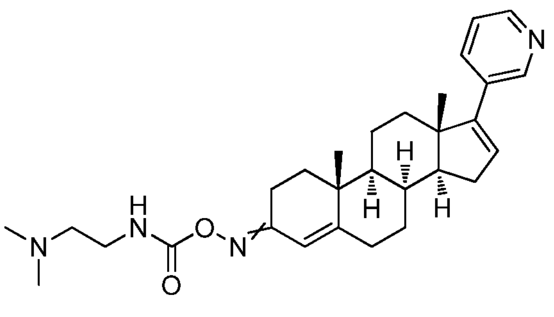

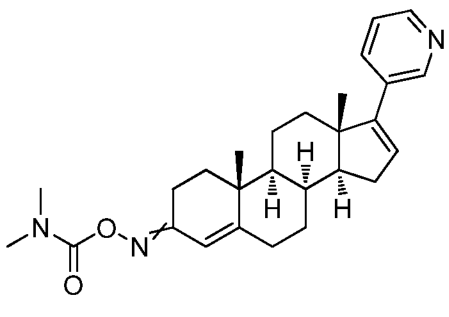

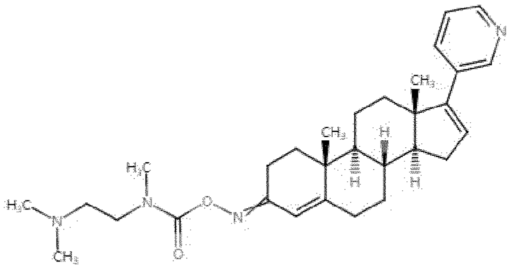

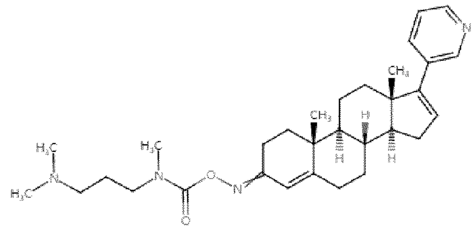

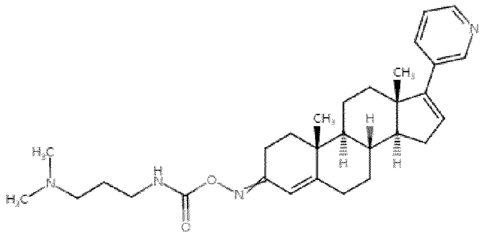

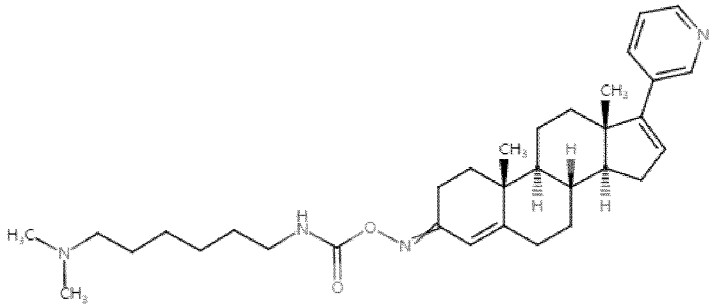

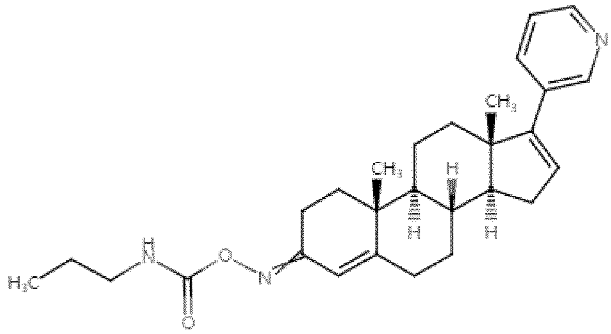

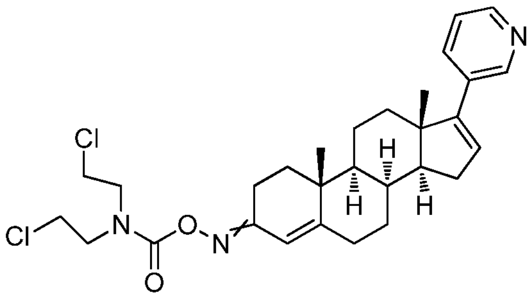

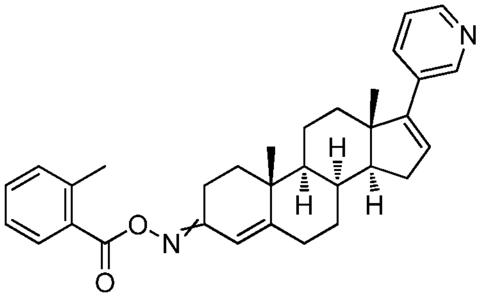

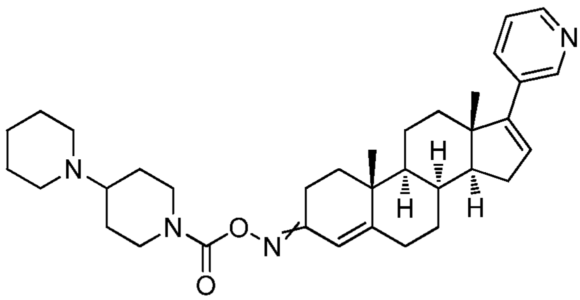

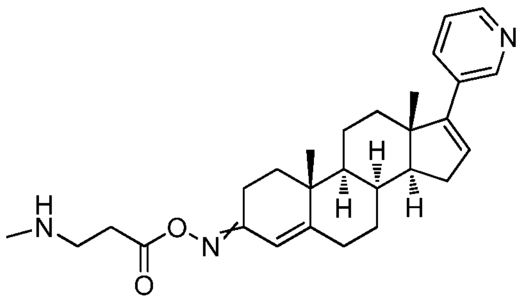

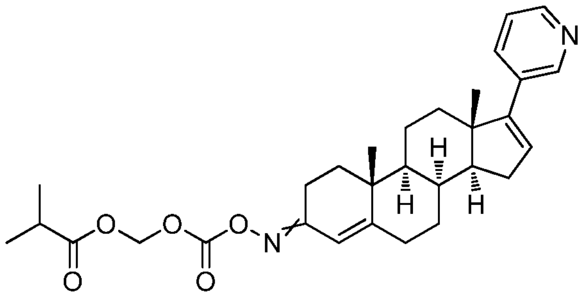
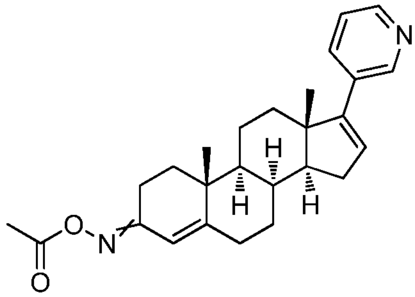

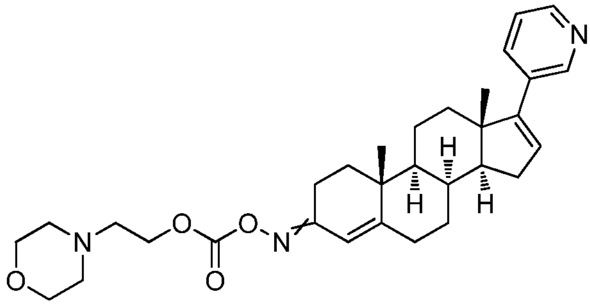
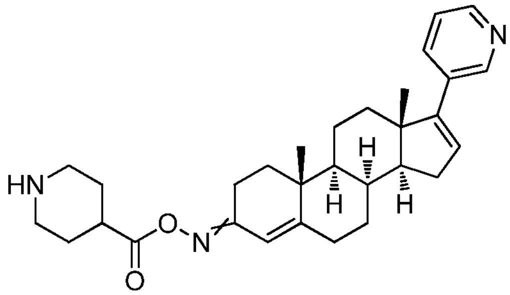

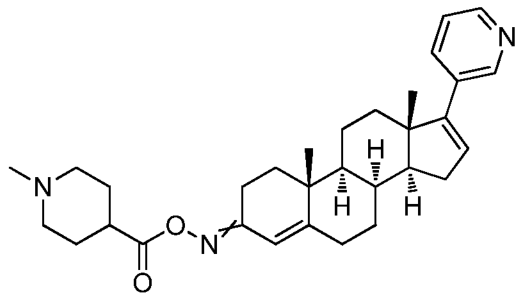
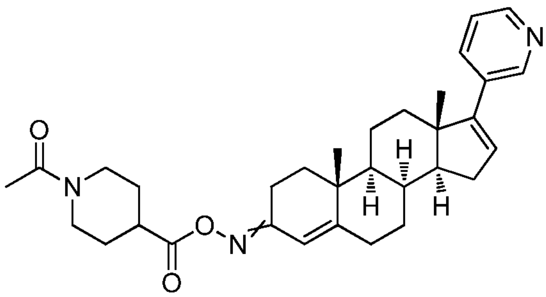

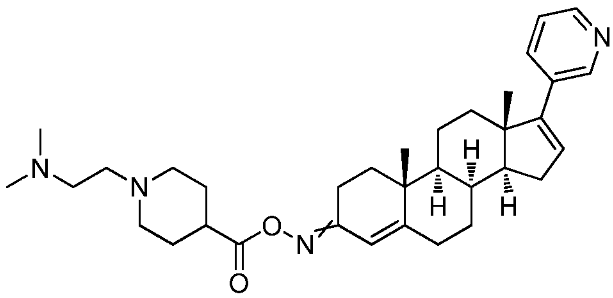
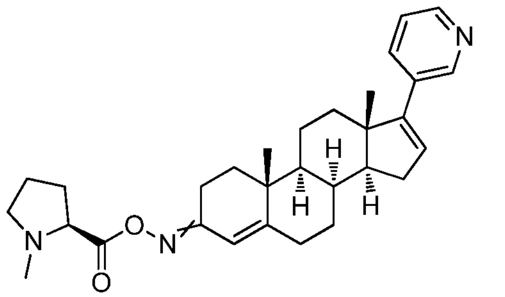

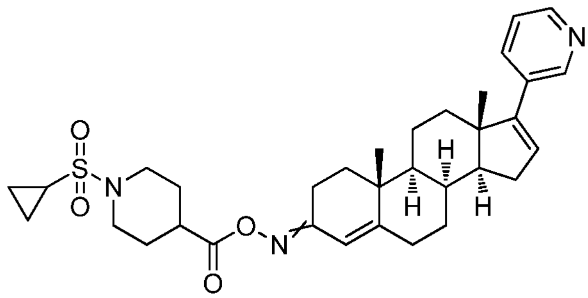
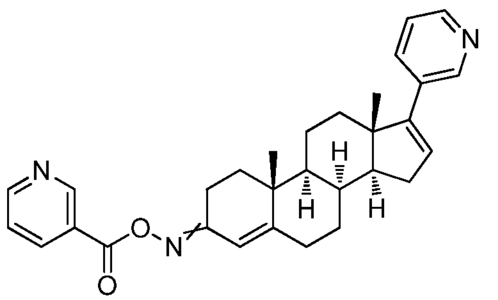

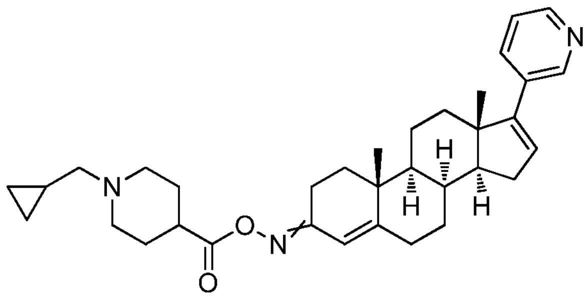

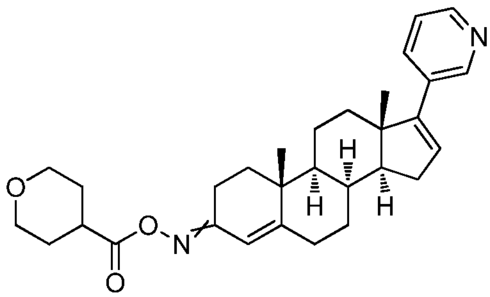

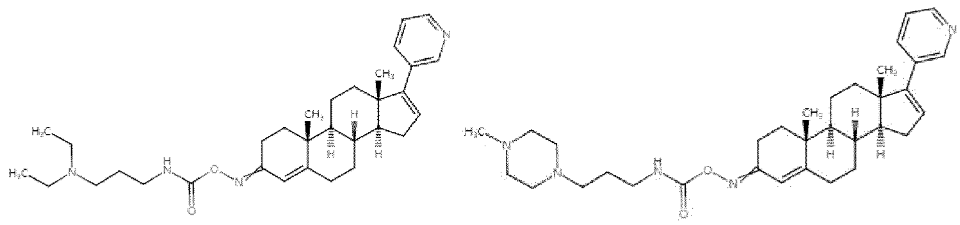

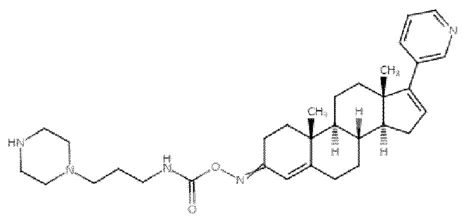

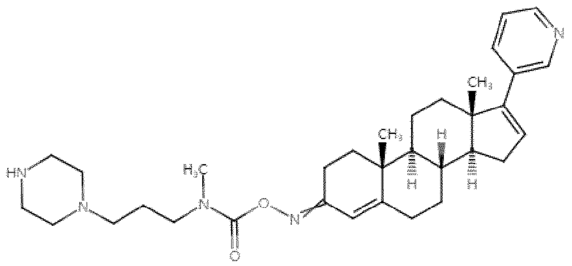

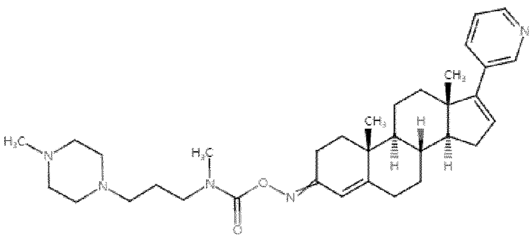

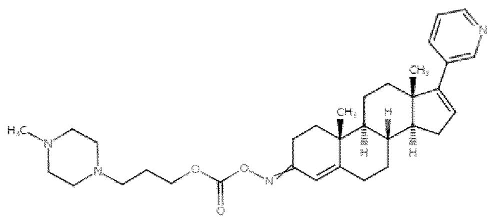

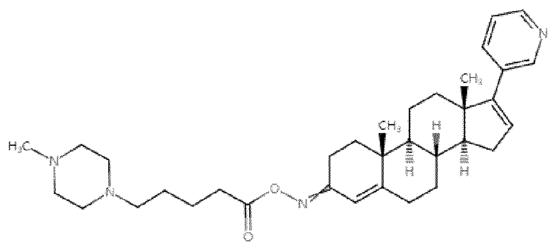

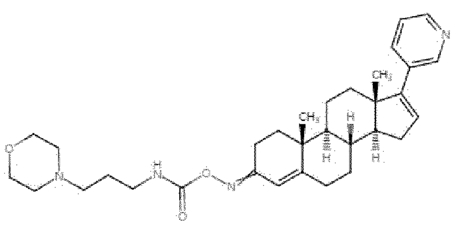

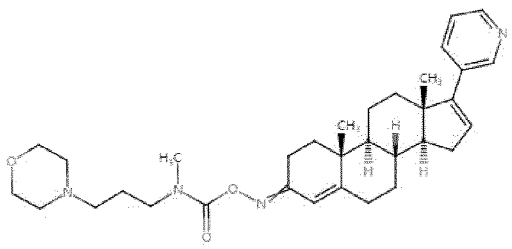

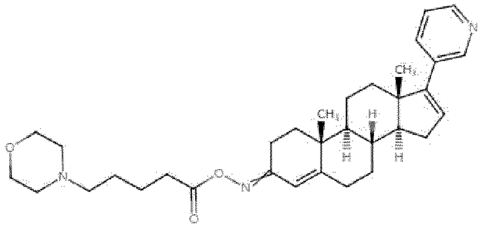

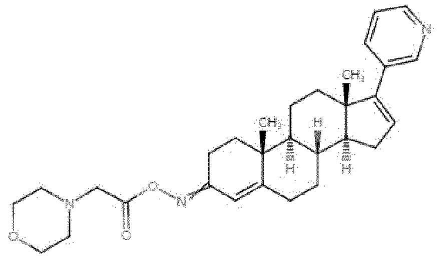

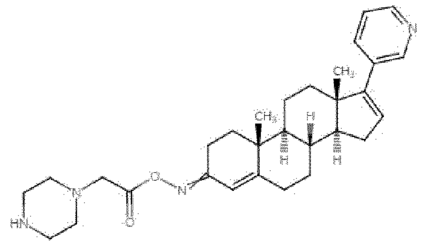

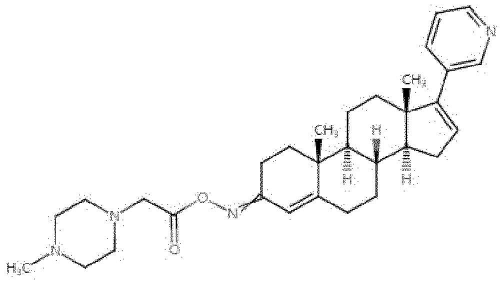

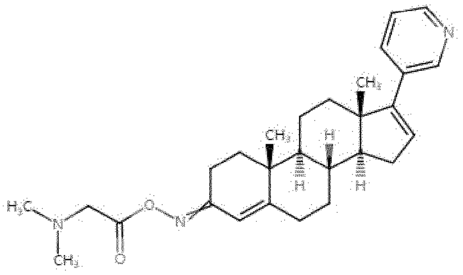

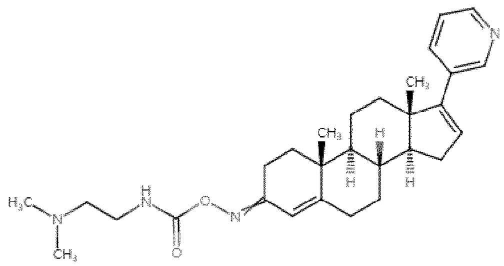

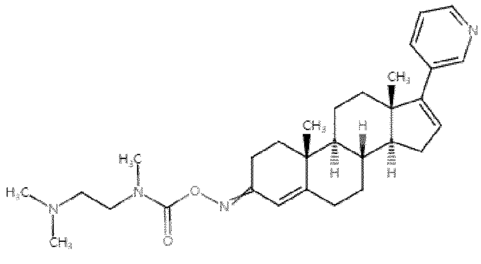

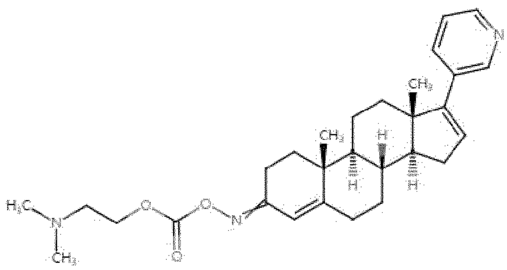

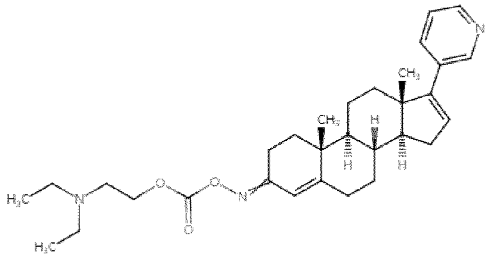

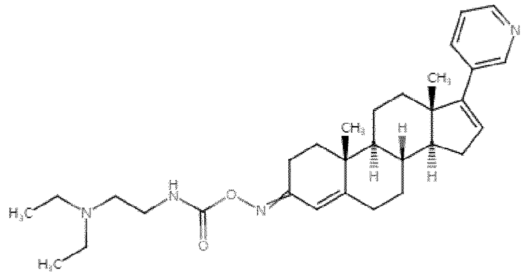

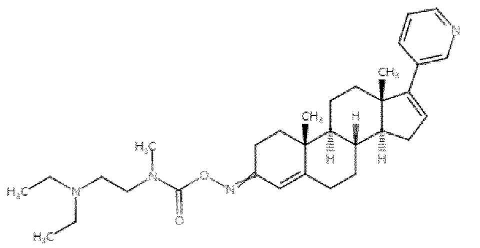

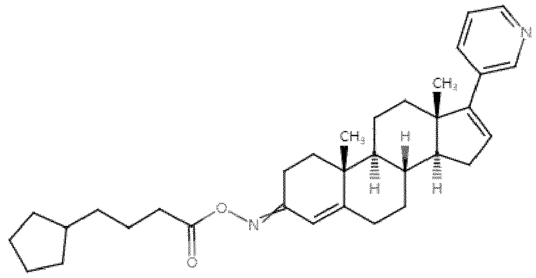

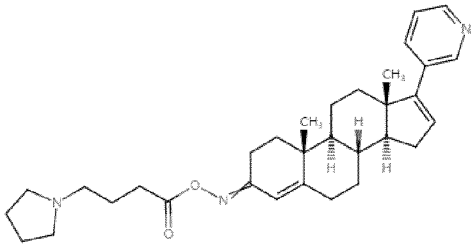

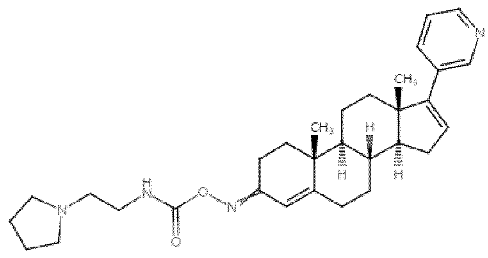

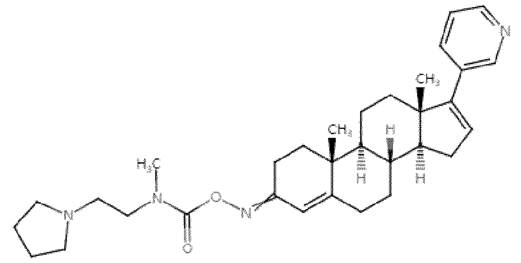

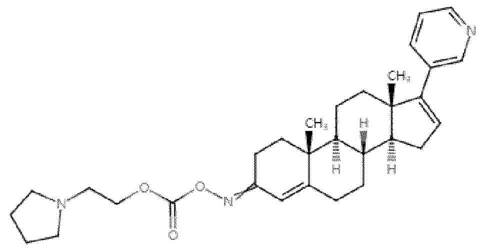

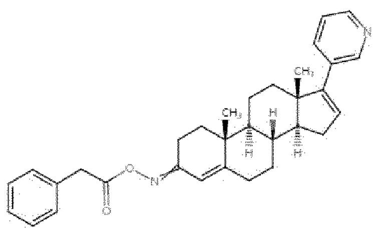

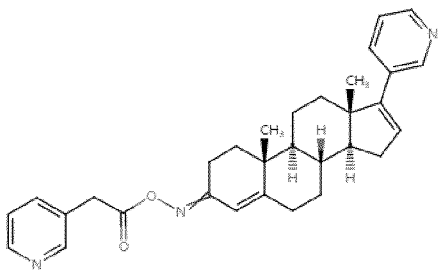

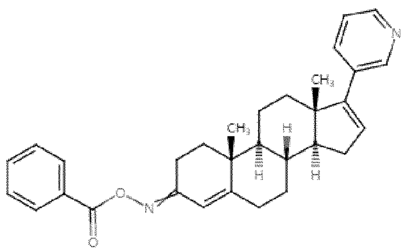

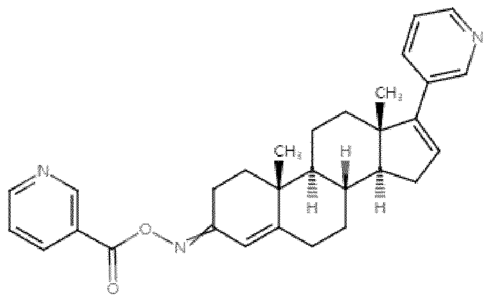

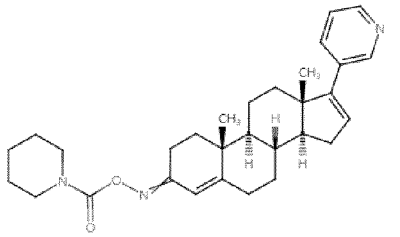

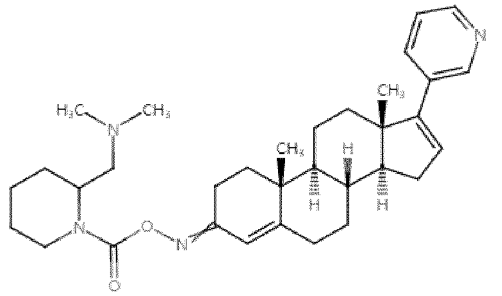

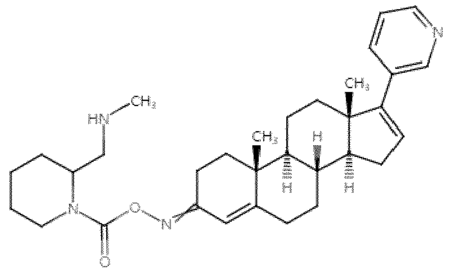

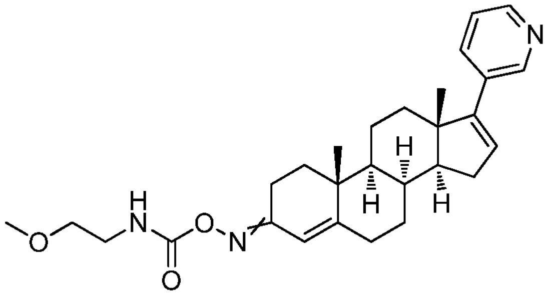

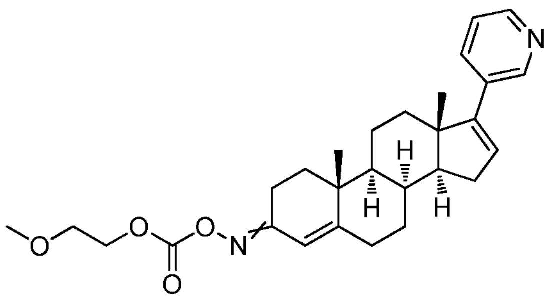

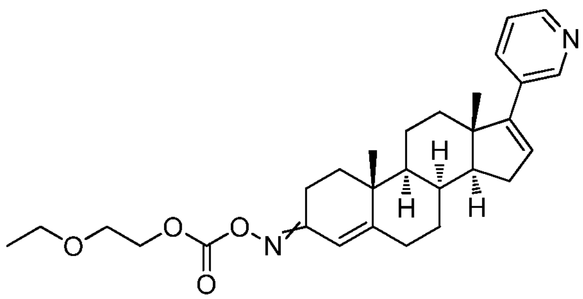

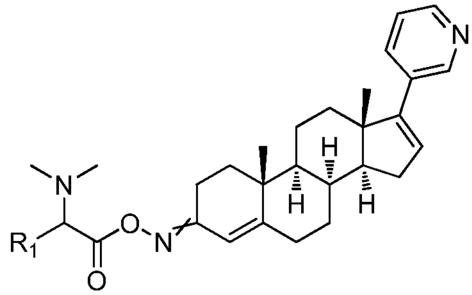

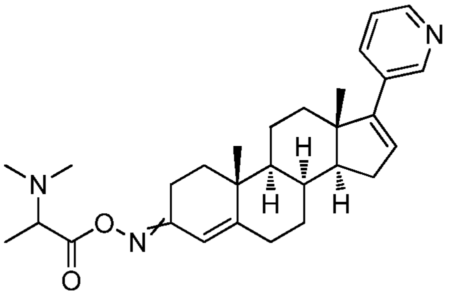

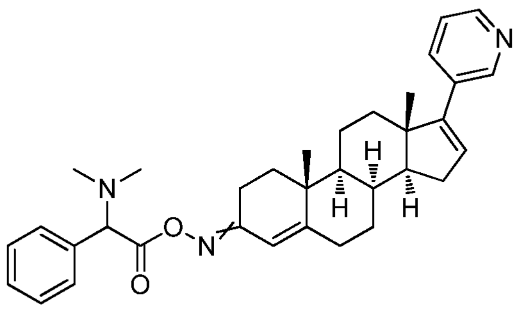

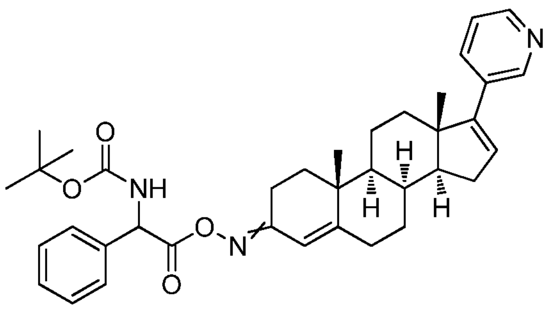

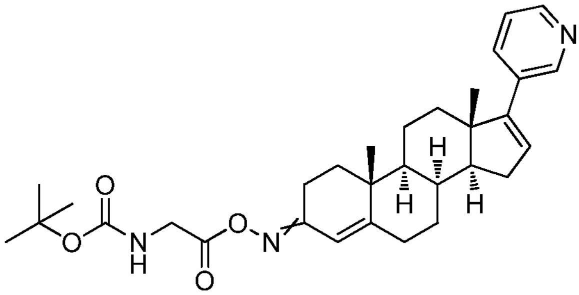

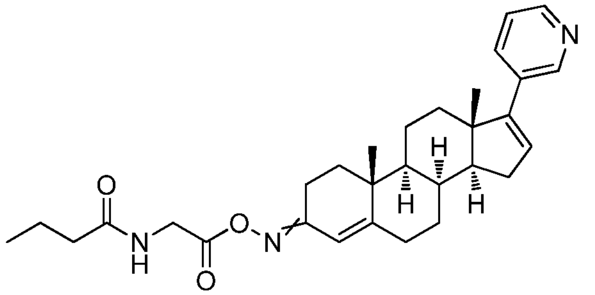

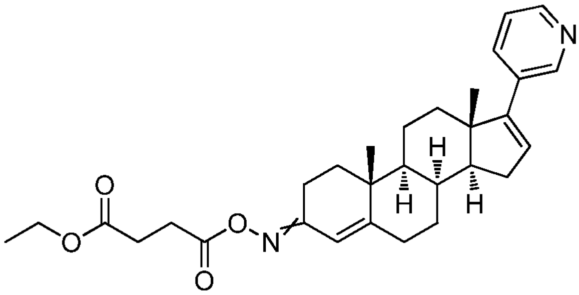

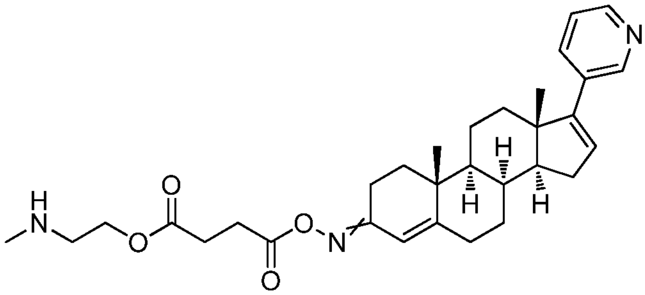

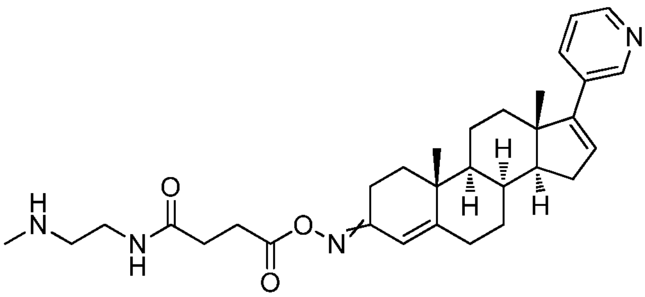

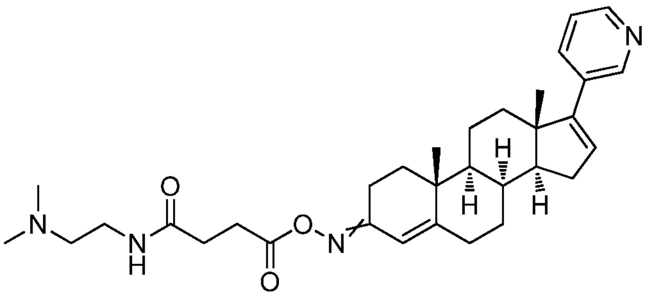

According to the reaction scheme shown in Figure 1, the abiraterone derivative shown in the above formula I is prepared by the method comprising the following steps:

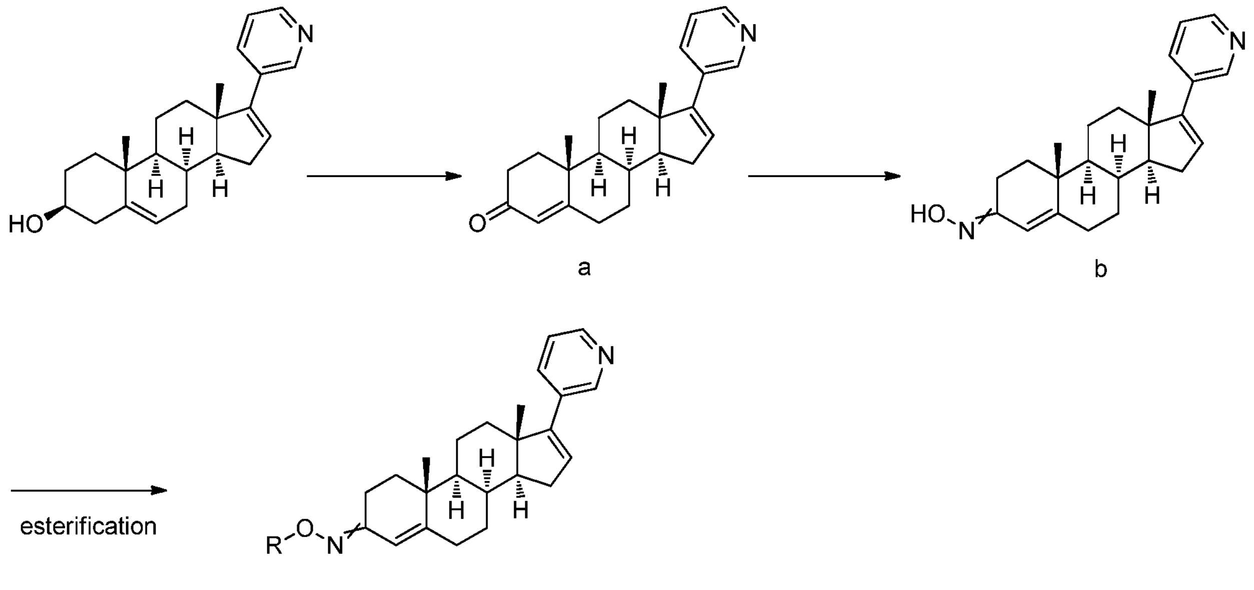


a

b

( I )

1) Compound a is obtained by oxidation reaction of abiraterone in the presence of aluminum isopropyl alcohol and cyclohexanone;

2)In alkaline conditions, condensation reaction of compound a with hydroxylamine hydrochloride was carried out to obtain compound b;

3)Compound b is directly esterified with the carbonyl compound of fragment R to obtain the abiraterone derivative shown in formula I; Alternatively, compound b forms an activator with carbonyldiimidazole, and then condenses with the corresponding alcohol and ammonia of fragment R to obtain the abiraterone derivative shown in formula I.

The use of the abiraterone derivative shown in the formula I in the following aspects also fall within the protection scope of the present invention:

1) Use for the preparation of eukaryotic tumor cell proliferation inhibitors;

2) Use for the preparation of a drug for the prevention and/or treatment of tumors.

The eukaryote is a mammal;

The tumor cells are cancer cells;

The cancer cells are prostate cancer cells;

The tumor is cancer; the cancer is prostate cancer.

The prostate cancer is metastatic castration-resistant prostate cancer after prior docetaxel chemotherapy.

The present invention also provides an eukaryotic tumor cell proliferation inhibitor , which comprises the abiraterone derivative shown in formula I.

The use of the compound shown in formula I for the preparation of a drug for treating acne also falls within the protection scope of the present invention.

The present invention also provides a drug for treating acne, which comprises the abiraterone derivative shown in formula I.

The present invention provides an abiraterone derivative and the preparation method therefor. Compared with abiraterone, the abiraterone derivative provided by the present invention exhibits relatively less abdominal distension and has better drug efficacy and tolerance. The pharmacokinetic experiment shows that the plasma concentration of M1 was significantly higher than that of abiraterone acetate after intragastric administration of equimolar doses, and M2 was detected in the metabolites, while M2 was not detected in metabolites of abiraterone acetate. It has a significantly better inhibitory effect on prostate cancer than that of abiraterone, and has less gastrointestinal side effects than abiraterone. The compound provided by the present invention also has the effect of treating acne.

## DESCRIPTION OF SPECIFIC EMBODIMENTS

Although the present invention will be described below through specific examples, it is not intended to be limited thereto.

The methods given in examples below are all conventional methods, unless otherwise stated. The materials and reagents are all commercially available unless otherwise stated.

Example 1

Synthesis of (8S,9S,10R,13S,14S)-10,13-Dimethyl-17-(pyridin-3-yl)-6,7,8,9,10,11,12,13,14,15-decahydro-1H-cyclopenteno[a]phenanthrene-3(2H)-one O-((2-(dimethylamino)ethyl)carbamoyl)oxime

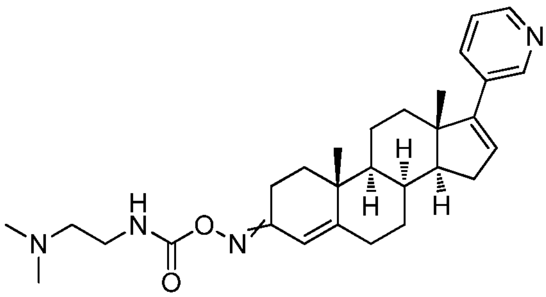

1)(8S,9S,10R,13S,14S)-10,13-Dimethyl-17-(pyridin-3-yl)-6,7,8,9,10,11,12,13,14,15-Decahydro-1H-cyclopenteno[a]phenanthrenyl-3(2H)-one

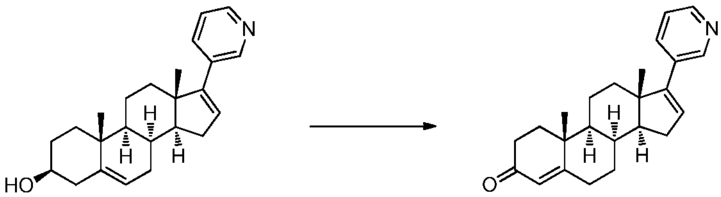

Abiraterone (35.0g, 100mmol, 1.0eq.), aluminum isopropoxide (20.5g, 100mmol, 1.0eq.), cyclohexanone (29.5g, 300mmol, 3.0eq.) were weighed into a round bottom reaction flask, and toluene (210mL) was added. Under the protection of nitrogen, and be heated at 100°C for 16h. The reaction solution was cooled, and added with NaCl aqueous solution, then the insoluble materials were filtered with celite, separated the organic layer, and purified by column chromatography to obtain a white solid (8S, 9S, 10R, 13S,14S)-10,13-dimethyl-17-(pyridine -3-yl)-6,7,8,9,10,11,12,13,14,15-decahydro-1H- cyclopenteno[a]phenanthrenyl-3(2H)-one (30.2g, 87.0%). 1H NMR(DMSO-d6 400MHz): δ 8.47(s, 1H), 8.33(d, 1H), 7.77-7.29(m, 2H), 5.96(m, 1H), 5.85(s, 1H), 2.99(m, 2H), 2.37-1.27(m, 21H). ESI-MS m/z: 348.1[M+H]+.

2)(8S,9S,10R,13S,14S)-10,13-Dimethyl-17-(pyridin-3-yl)-6,7,8,9,10,11,12,13,14,15-Decahydro-1H-cyclopenta [a]phenanthrenyl-3(2H)-ketoxime

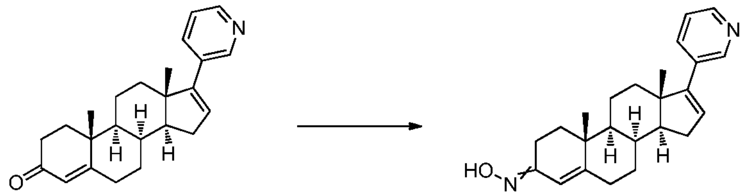

(8S, 9S, 10R, 13S, 14S)-10,13-dimethyl-17-(pyridin-3-yl)-C,7,8,9,10,11,12,13,14, 15-Decahydro-1H-cyclopenta[a]phenanthrenyl-3(2H)-one (30.0g, 86.5mmol, 1.0eq.) was weighted and dissolved in absolute ethanol (250mL), and then hydroxylamine hydrochloride (12.0 g, 173mmol, 2.0eq.), sodium acetate (21.3g, 260mmol, 3.0eq.) were added, and react at room temperature for 3h. Excess water was added to the reaction solution, and a white solid was precipitated. The crude product was filtered and recrystallized from ethanol to obtain (8S,9S,10R,13S,14S)-10,13-dimethyl-17-(pyridin-3-yl)-6,7,8,9,10,11,12 , 13,14,15-Decahydro-1H-cyclopenta[a]phenanthrenyl-3(2H)-ketoxime (25.0 g, 80.0%). 1H NMR (DMSO-d6 400MHz) δ 8.47(s, 1H), 8.33(d, 1H), 7.77-7.29(m, 2H), 5.96(m, 1H), 5.85(s, 1H), 4.62(s, 1H), 2.99(m, 2H), 2.37-1.27(m, 21H). ESI-MS m/z: 363.1 [M+H]+.

3)(8S,9S,10R,13S,14S)-10,13-Dimethyl-17-(pyridin-3-yl)-6,7,8,9,10,11,12,13,14,15-Decahydro-1H-cyclopenta[a]phenanthrene-3(2H)-one O-(1H-imidazole-1-carbonyl)oxime

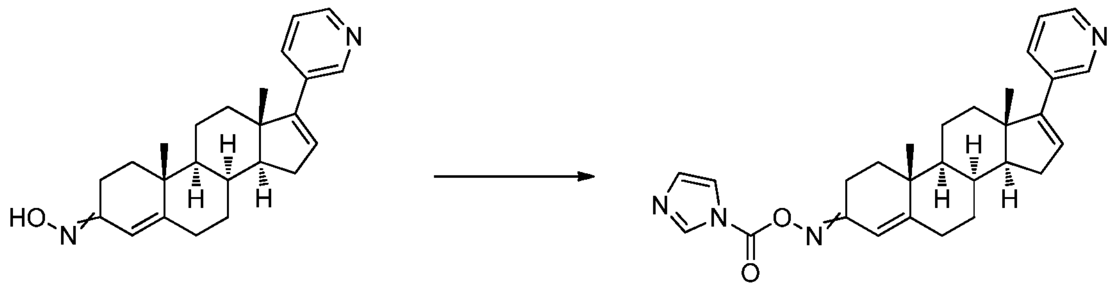

(8S,9S,10R,13S,14S)-10,13-Dimethyl-17-(pyridin-3-yl)-6,7,8,9,10,11,12,13,14,15-Decahydro-1H-cyclopenta[a]phenanthrenyl-3(2H)-ketoxime (2.0g, 5.52mmol, 1.0eq.) was dissolved in dichloromethane (20mL), and triethylamine ( 1.11 g, 11.0mmol. 2.0eq.), carbonyldiimidazole (1.78g, 11.0mmol. 2.0eq.) were added respectively, and the solution was stirred at room temperature for 8h. The reaction solution was extracted with water, the organic phase was concentrated, and (8S,9S,10R,13S,14S)-10,13-dimethyl-17-(pyridin-3-yl)-6,7,8,9,10,11,12,13,14,15-Decahydro-1H-cyclopenteno[a]phenanthrene-3(2H)-one O-(1H-imidazole-1-carbonyl)oxime was obtained by column chromatography (2.0g, 79.5 %). 1H NMR (DMSO-d6 400MHz): δ 8.47(s, 1H), 8.33(d, 1H), 8.14(s, 1H), 7.77-7.14(m, 4H), 5.96(m, 1H), 5.85(s, 1 H), 2.99(m, 2H), 2.37-1.27(m, 21H). ESI-MS m/z: 457.1 [M+H]+.

4)(8S,9S,10R,13S,14S)-10,13-Dimethyl-17-(pyridin-3-yl)-6,7,8,9,10,11,12,13,14,15-Decahydro-1H-cyclopenta[a]phenanthrene-3(2H)-one O-((2-(dimethylamino)ethyl)carbamoyl)oxime

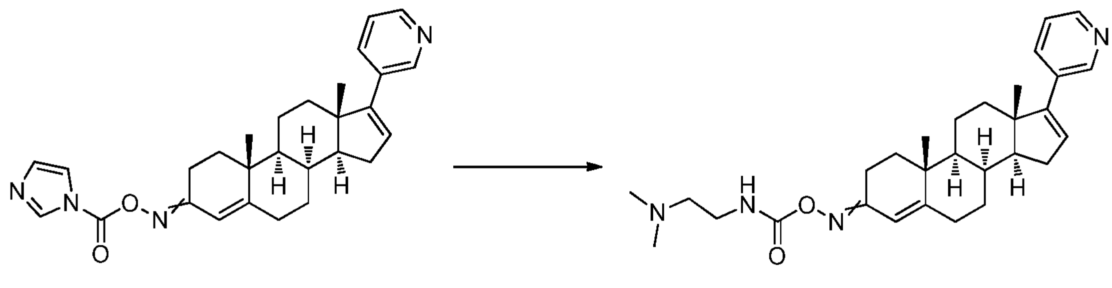

(8S,9S,10R,13S,14S)-10,13-Dimethyl-17-(pyridin-3-yl)-6,7,8,9,10,11,12,13,14,15-Decahydro-1H-cyclopenta[a]phenanthrene-3(2H)-one O-(1H-imidazole-1-carbonyl)oxime (2.0g, 4.39mmol, 1.0eq.) was dissolved in dichloromethane ( 10 mL), then N, N-dimethylethylenediamine (0.58 g, 6.58 mmol, 1.5 eq.) was added dropwise, and the solution was stirred at room temperature for 5 h. The reaction solution was concentrated and purified by column chromatography to obtain solid (8S,9S,10R,13S,14S)-10,13-dimethyl-17-(pyridin-3-yl)-6,7,8,9,10, 11,12,13,14, 15- Decahydro-IH-cyclopenteno[a]phenanthrene-3(2H)-one O-((2-(dimethylamino)ethyl)carbamoyl)oxime (1.5 g, 7.8%). 1H NMR(DMSO-d6 400MHz): δ 8.47(s, 1H), 8.33(d, 1H), 7.77-7.29(m, 2H), 5.96(m, 1H), 5.85(s, 1H), 3.28-3.26 (d, 4H), 2.36(s, 6H), 2.37-1.27(m, 23H). ESI-MS m/z: 477.1[M+H]+.

Example 2

Synthesis of (8S,9S,10R,13S,14S)-10,13-Dimethyl-17-(pyridin-3-yl)-6,7,8,9,10,11,12,13,14,15- decahydro-1H-cyclopenta[a]phenanthrene-3(2H)-one O-dimethylcarbamoyl oxime

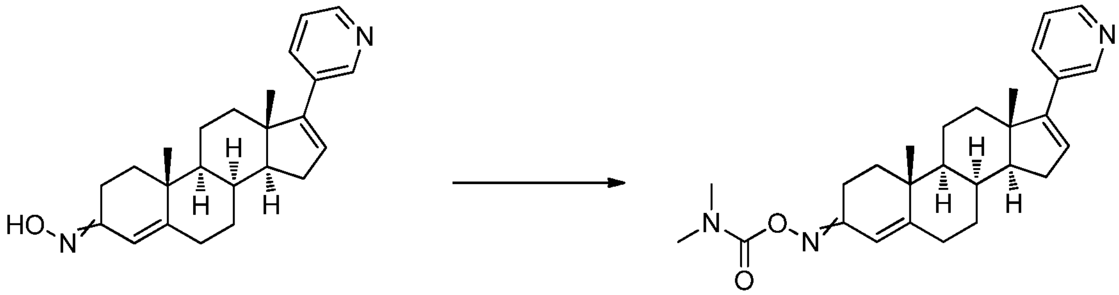

(8S,9S,10R,13S,14S)-10,13-Dimethyl-17-(pyridin-3-yl)-6,7,8,9,10,11,12,13,14,15-Decahydro-1H-cyclopenta[a]phenanthrenyl-3(2H)-ketoxime (2.0g, 5.52mmol, 1.0eq.), triethylamine (1.11g, 11.0mmol. 2.0eq.) respectively dissolve in dichloromethane (20 mL), and dimethylcarbamoyl chloride (1.19 g, 11.0 mmol, 2.0 eq.) was add ed dropwise, and react at room temperature for 5 h. The reaction solution was concentrated, and white solid (8S,9S,10R,13S,14S)-10,13-dimethyl-17-(pyridin-3-yl)-6,7,8,9,10,11,12,13,14,15-Decahydro-1H-cyclopenta[a]phenanthrene-3(2H)-one O-dimethylcarbamoyl oxime (2.0 g, 83.7%) was obtained by column chromatography gave. 1H NMR (DMSO-d6 400MHz): δ 8.47(s, 1H), 8.33(d, 1H), 7.77-7.29(m, 2H), 5.96(m, 1H), 5.85(s, 1H), 3.47(s, 6H), 2.37-1.27(m, 23H). ESI-MS m/z: 434.1[M+H]+..

Example 3

Synthesis of (8S,9S,10R,13S,14S)-10,13-Dimethyl-17-(pyridin-3-yl)-6,7,8,9,10,11,12,13,14,15- decahydro-1H-cyclopenta[a]phenanthrenyl-3(2H)-one O-(bis(2-chloroethyl)carbamoyl)oxime

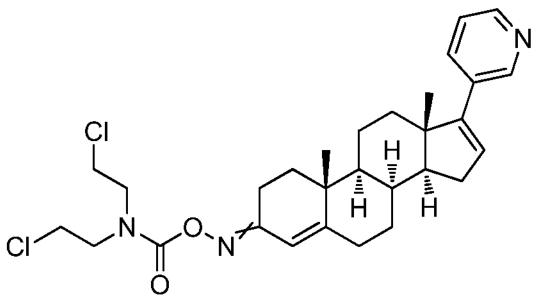

The synthetic method of Examples 1-2 was merged. After the esterification of bis(2-chloroethyl) carbamoyl chloride and (8S,9S,10R,13S,14S)-10,13-dimethyl-17-(pyridine-3-yl)-6,7,8,9,10,11,12,13,14,15-decahydro-1H- cyclopenteno[a]phenanthrenyl-3(2H)-ketoxime intermediate, a white solid (8S,9S,10R,13S,14S)-10,13-Dimethyl-17-(pyridin-3-yl)-6,7,8,9,10,11,12,13,14,15-Decahydro-1H-cyclopenta[a]phenanthrenyl-3(2H)-one O-(bis(2-chloroethyl)carbamoyl)oxime was obtained. 1H NMR (DMSO-d6 400MHz): δ 8.47(s, 1H), 8.33(d, 1H), 7.77-7.29(m, 2H), 5.96(m, 1H),

5.62(s, 1H), 3.64(m, 4H), 3.24(m, 4H), 2.37-1.27(m, 23H). ESI-MS m/z: 532.1[M+H]+.

Example 4

Synthesis of (8S,9S,10R,13S,14S)-10,13-Dimethyl-17-(pyridin-3-yl)-6,7,8,9,10,11,12,13,14,15- decahydro-1H-cyclopentene[a]phenanthrenyl-3(2H)-one O-([1,4'-bipiperidine]-1 '-carbonyl)oxime

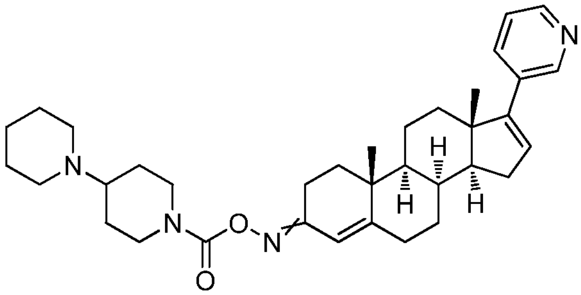

The synthetic method of Examples 1-2 was merged. After the esterification of [1,4'-bipiperidine]-1 '-carbonyl chloride and (8S,9S,10R,13S,14S)-10,13-dimethyl-17-(pyridin-3-yl)-6,7,8,9,10,11,12,13,14,15-decahydro-1H- cyclopenteno[a]phenanthrenyl-3(2H)-ketoxime intermediate, a white solid (8S,9S,10R,13S,14S)-10,13-dimethyl-17-(pyridin-3-yl)-6,7,8,9,10,11,12,13,14,15-Decahydro-1H-cyclopentene[a]phenanthrenyl-3(2H)-one O-([1,4'-bipiperidine]-1'-carbonyl)oxime was obtained. 1H NMR (DMSO-d6 400MHz): δ 8.47(s, 1H) ,8.33(d, 1H), 7.77-7.29(m, 2H), 5.96(m, 1H), 5.62(s, 1H), 3.39(m, 4H), 2.62-2.42(m, 5H), 2.37-1.27(m, 33H). ESI-MS m/z: 557.1[M+H]+.

Example 5

Synthesis of (8S,9S,10R,13S,14S)-10,13-Dimethyl-17-(pyridin-3-yl)-6,7,8,9,10,11,12,13,14,15- decahydro-1H-cyclopenta[a]phenanthrenyl-3(2H)-one O-(2-methylbenzoyl)oxime

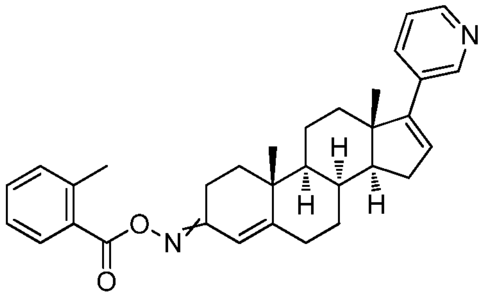

The synthetic method of Examples 1-2 was merged. After the esterification of o-toluyl chloride and (8S, 9S, 10R,13S,14S)-10,13-dimethyl-17-(pyridin-3-yl)-6,7,8,9,10,11,12,13,14,15-Decahydro-1H-cyclopenteno[a]phenanthrenyl-3(2H)-ketoxime intermediate, a white solid (8S, 9S,10R,13S,14S)-10,13-Dimethyl-17-(pyridin-3-yl)-6,7,8,9,10,11,12,13,14,15-decahydro-1H-Cyclopenteno[a]phenanthrenyl-3(2H)-one O-(2-methylbenzoyl)oxime was obtained. 1H NMR(DMSO-d6 400MHz): δ 8.47(s, 1H), 8.33(d, 1H), 7.88-7.29(m, 6H), 5.96(m, 1H), 5.62(s, 1H), 2.37-1.27 (m, 26H). ESI-MS m/z: 481.1[M+H]+.

Example 6

Synthesis of (8S,9S,10R,13S,14S)-10,13-Dimethyl-17-(pyridin-3-yl)-6,7,8,9,10,11,12,13,14,15- decahydro-1H-cyclopenta[a]phenanthrenyl-3(2H)-one O-(3-(methylamino)propionyl)oxime

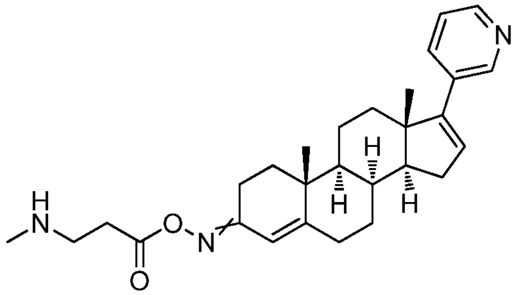

The synthetic method of Examples 1-2 was merged. After the esterification of 3-methylaminopropionic acid and (8S,9S,10R,13S,14S)-10,13-dimethyl-17-(pyridin-3-yl)-6,7,8,9,10,11,12,13,14,15-Decahydro-1H- cyclopenteno[a]phenanthrenyl -3(2H)-ketoxime intermediate, a white solid ( 8S,9S,10R,13S,14S)-10,13-Dimethyl-17-(pyridin-3-yl)-6,7,8,9, 10,11,12,13,14,15-decahydro -1H -cyclopenta[a]phenanthrenyl-3(2H)-one O-(3-(methylamino)propionyl)oxime was obtained by deprotection. 1H NMR (DMSO-d6 400MHz) : δ 8.47(s, 1H), 8.33(d, 1H), 7.77-7.29(m, 2H), 5.96(m, 1H), 5.62(s, 1H), 3.62(s, 3H), 2.83-2.63(m, 4H), 2.37-1.27(m, 23H). ESI-MS m/z: 448.1[M+H]+.

Example 7

Synthesis of (8S,9S,10R,13S,14S)-10,13-Dimethyl-17-(pyridin-3-yl)-6,7,8,9,10,11,12,13,14,15- decahydro-1H-cyclopenta[a]phenanthrenyl-3(2H)-one O-acetyloxime

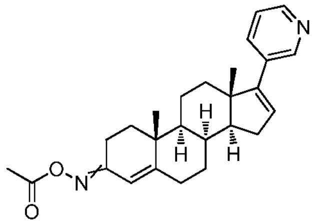

The synthetic method of Examples 1-2 was merged. After the esterification of acetic anhydride and (8S, 9S, 10R, 13S, 14S)-10,13-dimethyl-17-(pyridin-3-yl)-6,7,8 ,9,10,11,12,13,14,15-Decahydro-1H-cyclopenteno[a] phenanthrenyl-3(2H)-ketoxime intermediate, a white solid (8S,9S, 10R,13S,14S)-10,13-Dimethyl-17 -(pyridin-3-yl)-6,7,8,9,10,11,12,13,14,15 -decahydro-1H- Cyclopenteno[a]phenanthrenyl-3(2H)-one O-acetyl oxime was obtained by deprotection. 1H NMR(DMSO-d6 400MHz): δ 8.47(s, 1H), 8.33(d, 1H), 7.77-7.29(m, 2H), 5.96(m, 1H), 5.62(s, 1H), 2.37-1.27 (m, 26H). ESI-MS m/z: 405.1[M+H]+.

Example 8

Synthesis of ((((((8S,9S,10R,13S,14S)-10,13-dimethyl-17-(pyridin-3-yl)-7,8,9,11,12,13,14,15-octahydro-1H-cyclopenta [a]phenanthrenyl-3(2H,6H,10H)-ylidene)amino)oxy)carbonyl)oxy)methyl isobutyrate

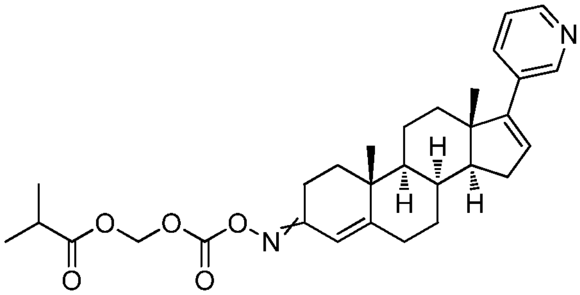

The synthetic method of Examples 1-2 was merged. After the esterification of chloromethyl chloroformate and (8S, 9S, 10R, 13S, 14S)-10,13-dimethyl-17-(pyridin-3-yl)-6, 7,8,9,10,11,12,13,14,15-Decahydro- 1H-cyclopenta[a]phenanthrenyl-3(2H)-ketoxime intermediate, a white solid (((((8S,9S,10R,13S,14S)- 10,13-dimethyl-17-(pyridin-3-yl)-7,8,9,11,12,13,14,15-Octahydro-1H-cyclopenta[a]phenanthrenyl-3(2H,6H,10H)-ylidene)amino)oxy)carbonyl)oxy)methyl isobutyrate was obtained by esterification with isobutyric acid. 1H NMR (DMSO-d6 400MHz): δ 8.47(s, 1H), 8.33(d, 1H), 7.70-7.29(m, 2H), 6.95(s, 1H), 5.96(m, 1H), 5.62(s, 1H), 2.67-1.14(m, 30H). ESI-MS m/z: 507.1[M+H]+.

Example 9

Synthesis of (8S,9S,10R,13S,14S)-10,13-Dimethyl-17-(pyridin-3-yl)-6,7,8,9,10,11,12,13,14,15-decahydro-1H-cyclopenta[a]phenanthrenyl-3(2H)-one O-((2-morpholineethoxycarbonyl)oxime

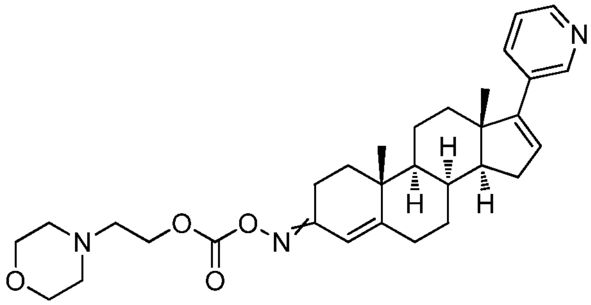

The synthetic method of Examples 1-2 was merged. 2-morpholine ethanol and (8S, 9S, 10R, 13S, 14S)-10,13-dimethyl-17-(pyridin-3-yl)-6, 7,8,9,10,11,12,13,14,15-Decahydro-1H-cyclopenta[a]phenanthrenyl-3(2H)-ketoxime intermediate were reacted in a triphosgene environment to form a carbonate and (8S ,9S,10R,13S,14S)-10,13-Dimethyl-17-(pyridin-3-yl)-6,7,8,9,10,11,12,13,14,15-decahydro -1H-Cyclopenteno[a]phenanthrenyl-3(2H)-one O-((2-morpholineethoxycarbonyl)oxime was obtained. 1H NMR(DMSO-d6 400MHz): δ 8.47(s, 1H), 8.33(d, 1H), 7.70-7.29(m, 2H), 5.96(m, 1H), 5.62(s, 1H), 4.43(m, 2H), 3.65(m, 4H), 2.97(m, 2H ), 2.36(m, 4H), 2.12-1.12(m, 23H) .ESI-MS m/z: 520.1[M+H]+.

Example 10

Synthesis of (8S,9S,10R,13S,14S)-10,13-Dimethyl-17-(pyridin-3-yl)-6,7,8,9,10,11,12,13,14,15-decahydro-1H-cyclopenta[a]phenanthrenyl-3(2H)-one O-piperidine-4-carbonyl oxime

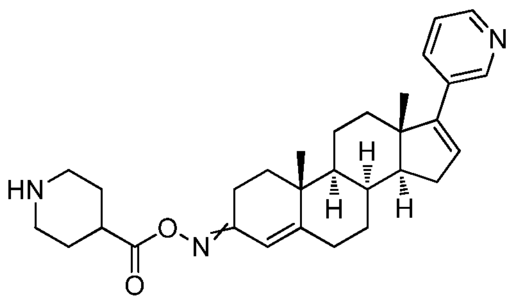

The synthetic method of Examples 1-2 was merged. After the esterification of 4-piperidinecarboxylic acid and (8S, 9S, 10R, 13S, 14S)-10,13-dimethyl-17-(pyridin-3-yl)-6, 7,8,9, 10, 11,12,13,14,15-Decahydro-1H-cyclopenta[a]phenanthrenyl-3(2H)-ketoxime intermediate, a white solid (8S,9S ,10R,13S,14S)-10,13-Dimethyl-17-(pyridin-3-yl)-6,7,8,9,10,11,12,13,14,15-decahydro-1H -Cyclopenteno[a]phenanthrenyl-3(2H)-one O-piperidine-4-carbonyl oxime was obtained. 1H NMR(DMSO-d6 400MHz):δ 8.47(s, 1H), 8.33(d, 1H), 7.70-7.29(m, 2H), 5.69(m, 1H), 5.62(s, 1H), 2.79(m, 4H), 2.33-1.27(m, 28H). ESI-MS m/z: 474.1[M+H]+.

Example 11

Synthesis of (8S,9S,10R,13S,14S)-10,13-Dimethyl-17-(pyridin-3-yl)-6,7,8,9,10,11,12,13,14,15- decahydro-1H-cyclopenta[a]phenanthrenyl-3(2H)-one O-(1-methylpiperidine-4-carbonyl)oxime

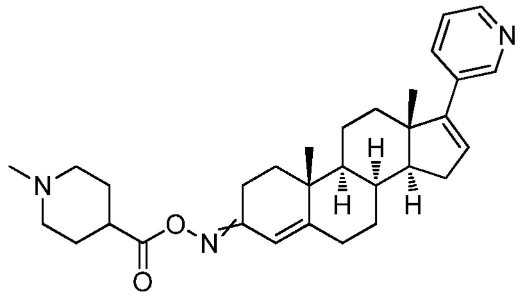

The synthetic method of Examples 1-2 was merged. After the esterification of 1-methylpiperidine-4-carboxylic acid and (8S, 9S, 10R, 13S, 14S)-10,13-dimethyl-17-(pyridin-3-yl)-6, 7,8,9,10,11,12,13,14,15-Decahydro-1H-cyclopenta[a]phenanthrenyl-3(2H)-ketoxime intermediate, a white solid (8S,9S,10R,13S,14S)-10,13-Dimethyl-17-(pyridin-3-yl)-6,7,8,9,10,11,12,13,14,15-Decahydro-1H-cyclopenta[a]phenanthrenyl-3(2H)-one O-(1-methylpiperidine-4-carbonyl)oxime. 1H NMR(DMSO-d6 400MHz): δ 8.47(s, 1H), 8.33(d, 1H), 7.70-7.29(m, 2H), 5.96(m, 1H), 5.62(s, 1H), 2.79(m, 4H), 2.33-1.27(m, 31H). ESI-MS m/z: 488.1[M+H]+.

Example 12

Synthesis of 1-(4-(((((8S,9S,10R,13S,14S)-10,13-dimethyl-17-(pyridin-3-yl)-7,8,9,11,12,13, 14,15-octahydro-1H-cyclopenteno[a]phenanthrenyl-3(2H,6H,10H)-ylidene)amino)oxy)carbonyl)piperidin-1-yl)ethanone

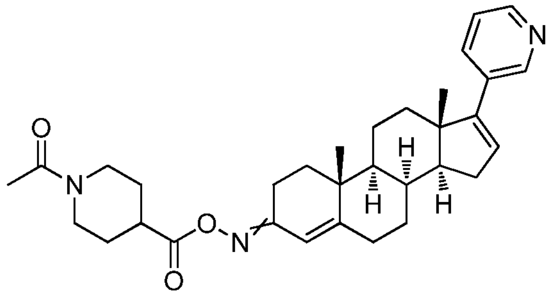

The synthetic method of Examples 1-2 was merged. After the esterification of 1-acetylpiperidine-4-carboxylic acid and (8S,9S,10R,13S,14S)-10,13-dimethyl-17-(pyridine-3-base)-6,7,8, 9,10,11,12,13,14,15-decahydro-1H-cyclopenteno[a]phenanthrenyl-3(2H)-ketoxime intermediate, a white Solid 1-(4-(((((8S,9S,10R,13S,14S)-10,13-Dimethyl-17-(pyridin-3-yl)-7,8,9,11,12,13,14,15-Octahydro-1H-cyclopenta[a]phenanthrenyl-3(2H,6H,10H)-ylidene)amino)oxy)carbonyl)piperidin-1-yl)ethanone was obtained. 1H NMR (DMSO-d6 400MHz): δ 8.47(s, 1H), 8.33(d, 1H), 7.70-7.29(m, 2H), 5.96(m, 1H), 5.62(s, 1H), 3.39(m, 4H), 2.33-1.27(m, 31H). ESI-MS m/z: 516.1[M+H]+.

Example 13

Synthesis of (8S,9S,10R,13S,14S)-10,13-Dimethyl-17-(pyridin-3-yl)-6,7,8,9,10,11,12,13,14,15-decahydro-1H- cyclopenta[a]phenanthrenyl-3(2H)-one 0-(1-(2-(dimethylamino)ethyl)piperidine-4-carbonyl)oxime

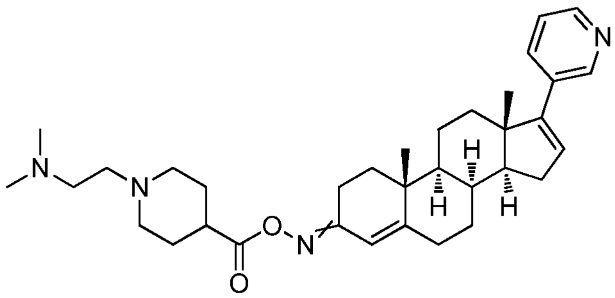

The synthetic method of Examples 1-2 was merged. After the esterification of 1-(2-(dimethylamino)ethyl)piperidine-4-carboxylic acid and (8S,9S,10R,13S,14S)-10,13-dimethyl-17-(pyridine-3-base)-6,7,8,9,10,11,12,13,14,15-decahydro-1H-cyclopenteno[a]phenanthrenyl-3(2H)-ketoxime intermediate, a white solid (8S,9S,10R,13S,14S)-10,13-dimethyl-17-(pyridin-3-yl)-6,7,8,9,10,11,12,13,14,15-Decahydro-1H-cyclopenteno[a]phenanthrenyl-3(2H)-one 0-(1-(2-(dimethylamino)ethyl)piperidine- 4-carbonyl)oxime was obtained. 1H NMR(DMSO-d6 400MHz): δ 8.47(s, 1H), 8.33(d, 1H), 7.70-7.29(m, 2H), 5.96(m, 1H), 5.62(s, 1H), 2.66(m, 6H), 2.41-2.37(m, 8H), 2.36-1.27(m, 28H). ESI-MS m/z: 545.1[M+H]+.

Example 14

Synthesis of (8S,9S,10R,13S,14S)-10,13-Dimethyl-17-(pyridin-3-yl)-6,7,8,9,10,11,12,13,14,15- decahydro-1H-cyclopenta[a]phenanthrenyl-3(2H)-one O-(1-(cyclopropanesulfonyl)piperidine-4-carbonyl)oxime

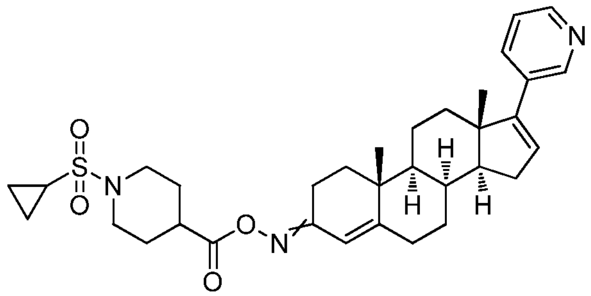

The synthetic method of Examples 1-2 was merged. After the esterification of 1-(cyclopropanesulfonyl) piperidine-4-carboxylic acid and (8S, 9S, 10R, 13S, 14S)-10,13-dimethyl-17-(Pyridin-3-yl)- 6,7,8,9,10,11,12,13,14,15-decahydro-1H-cyclopenta[a]phenanthrenyl-3(2H)-ketoxime intermediate, a white solid (8S,9S,10R,13S,14S)-10,13-dimethyl-17-(pyridin-3-yl)-6,7,8,9,10,11,12,13,14,15-Decahydro-1H-cyclopenta[a]phenanthrenyl-3(2H)-one O-(1-(cyclopropanesulfonyl)piperidine-4-carbonyl)oxime was obtained. 1H NMR (DMSO-d6 400MHz): δ 8.47(s, 1H), 8.33(d, 1H), 7.70-7.29(m, 2H), 5.96(m, 1H), 5.62(s, 1H), 2.91(m, 4H), 2.36-1.27(m, 33H). ESI-MS m/z: 578.1[M+H]+.

Example 15

Synthesis of (8S,9S,10R,13S,14S)-10,13-Dimethyl-17-(pyridin-3-yl)-6,7,8,9,10,11,12,13,14,15- decahydro-1H-cyclopenta[a]phenanthrenyl-3(2H)-one O-(1-(cyclopropanemethyl)piperidine-4-carbonyl)oxime

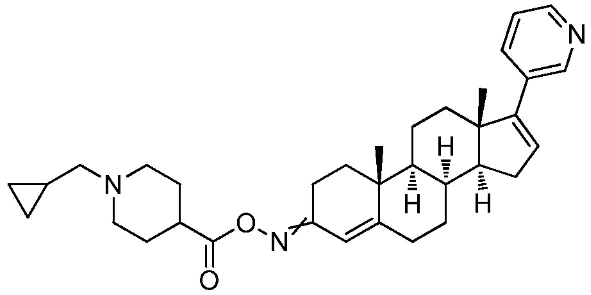

The synthetic method of Examples 1-2 was merged. After the esterification of 1-(cyclopropanemethyl) piperidine-4-carboxylic acid and (8S,9S,10R,13S,14S)-10,13-dimethyl-17-(Pyridin-3-yl)- 6,7,8,9,10,11,12,13,14,15-decahydro-1H-cyclopenta[a]phenanthrenyl-3(2H)-ketoxime intermediate, a white solid (8S,9S,10R,13S,14S)-10,13-dimethyl-17-(pyridin-3-yl)-6,7,8,9,10,11,12,13,14,15-Decahydro-1H-cyclopenta[a]phenanthrenyl-3(2H)-one O-(1-(cyclopropanemethyl)piperidine-4-carbonyl)oxime was obtained. 1H NMR(DMSO-d6 400MHz): δ 8.47(s, 1H), 8.33(d, 1H), 7.70-7.29(m, 2H), 5.96(m, 1H), 5.62(s, 1H), 2.41(m, 4H), 2.36-1.27(m, 35H). ESI-MS m/z: 528.1[M+H]+.

Example 16

Synthesis of (8S,9S,10R,13S,14S)-10,13-Dimethyl-17-(pyridin-3-yl)-6,7,8,9,10,11,12,13,14,15- decahydro-1H-cyclopentη[a]phenanthrenyl-3(2H)-one O-((S)-1-methylpyrrolidine-2-carbonyl)oxime

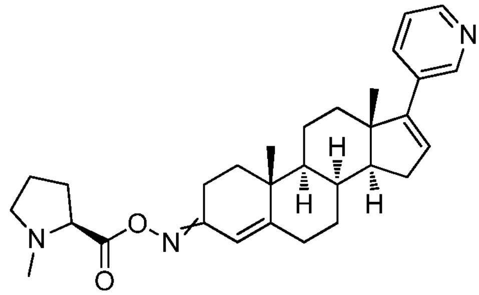

The synthetic method of Examples 1-2 was merged. After the esterification of (S)-1-methylpyrrolidine-2-carboxylic acid and (8S, 9S, 10R, 13S, 14S)-10,13-dimethyl-17-(Pyridin-3-yl)-6,7,8,9,10,11,12,13,14,15-decahydro-1H-cyclopenta[a]phenanthrenyl-3(2H)-ketoxime intermediate, a white solid (8S,9S,10R,13S,14S)-10,13- dimethyl-17-(pyridin-3-yl)-6,7,8,9,10,11,12,13,14,15-Decahydro-1H-cyclopenta[a]phenanthrenyl-3(2H)-one O-((S)-1-methylpyrrolidine-2-carbonyl)oxime was obtained. 1H NMR(DMSO-d6 400MHz): δ 8.47(s, 1H), 8.33(d, 1H), 7.70-7.29(m, 2H), 5.96(m, 1H), 5.62(s, 1H), 3.08(m, 1H), 2.36-1.27(m, 32H). ESI-MS m/z: 474.1[M+H]+.

Example 17

Synthesis of (8S,9S,10R,13S,14S)-10,13-Dimethyl-17-(pyridin-3-yl)-6,7,8,9,10,11,12,13,14,15- decahydro-1H-cyclopenta[a]phenanthrenyl-3(2H)-one O-tetrahydro-2H-pyran-4-carbonyl oxime

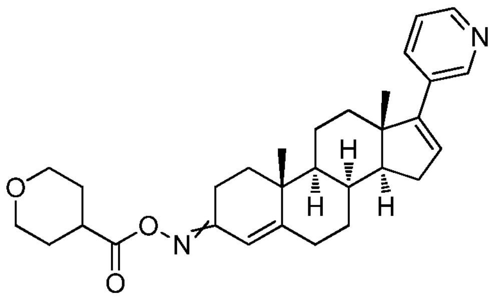

The synthetic method of Examples 1-2 was merged. After the esterification of tetrahydropyran-4-carboxylic acid and (8S, 9S, 10R, 13S, 14S)-10,13-dimethyl-17-(pyridin-3-yl) -6,7,8,9,10,11,12,13,14,15-decahydro-1H-cyclopenta[a]phenanthreny 1-3(2H)-ketoxime intermediate, a white solid (8S,9S,10R,13S,14S)-10,13-Dimethyl-17-(pyridin-3-yl)-6,7,8,9,10,11,12,13,14,15-10 Hydrogen-1H-cyclopenta[a]phenanthrenyl-3(2H)-one O-tetrahydro-2H-pyran-4-carbonyl oxime. 1H NMR(DMSO-d6 400MHz): δ 8.47(s, 1H), 8.33(d, 1H), 7.70-7.29(m, 2H), 5.96(m, 1H), 5.62(s, 1H), 3.55(m, 4H), 2.36-1.27(m, 28H). ESI-MS m/z: 475.1[M+H]+.

Example 18

Synthesis of (8S,9S,10R,13S,14S)-10,13-Dimethyl-17-(pyridin-3-yl)-6,7,8,9,10,11,12,13,14,15- Decahydro-1H-Cyclopenteno[a]phenanthrenyl-3(2H)-one O-Nicotinoxime

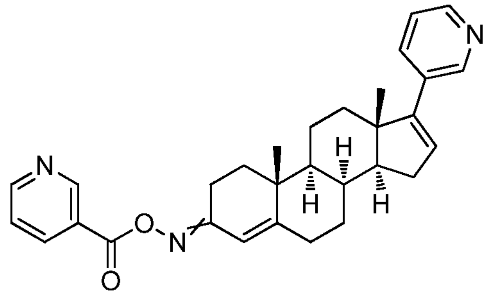

The synthetic method of Examples 1-2 was merged. After the esterification of nicotinic acid and (8S, 9S, 10R, 13S,14S)-10,13-dimethyl-17-(pyridin-3-yl)-6,7,8 ,9,10,11,12,13,14,15-Decηhydro-1H-cyclopenteno [a]phenanthrenyl-3 (2H)-ketoxime intermediate, a white solid (8S,9S,10R, 13S,14S)-10,13-Dimethyl-17-(pyridin-3-yl)-6,7,8,9,10,11,12,13,14,15- decahydro-1H-cyclopenta Ekeno[a]phenanthrenyl-3(2H)-one O-nicotinoyl oxime. 1H NMR (DMSO-d6 400MHz): δ 8.88(s, 1H), 8.65(d, 1H), 8.47(s, 1H), 8.33(d, 1H), 7.70-7.29(m, 4H), 5.96(m, 1H), 5.62(s, 1H), 2.36-1.27(m, 23H). ESI-MS m/z: 468.1 [M+H]+.

Example 19: Prostate tumor pharmacodynamics experiment

Test the inhibitory effects of the compounds of ZONK1901 series (compounds of the present invention) and ZONK1901-10 (abiraterone acetate) on the growth of human prostate cancer cell VCaP nude mouse transplanted tumor model in vivo.

Test sample: compound (B) of Example 1, compound (C) of Example 2, compound (D) of Example 3, Example 4 (E), Example 5 (F), Example 6 (G), Example 7 (H) and ZONK1901-19, ZONK1901-10 (I) (abiraterone acetate)

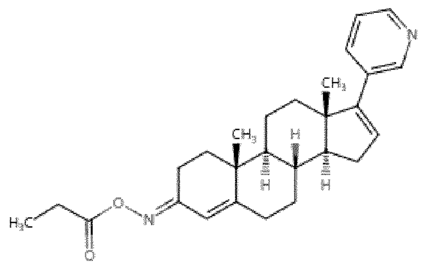

ZONK1901-19 (Group J).

Reagent

DMEM culture solution; fetal bovine serum; trypsin; penicillin-streptomycin double antibody; dimethyl sulfoxide (DMSO); TPGS; Matrigel; PBS (pH7.4, 0.01M).

Male BALB/c nude mice (number: 90; age: 6-7 weeks) purchased from Beijing Vital River Laboratory Animal Technology Co., Ltd., were raised in the SPF animal room of Suzhou Shengsu New Drug Development Co., Ltd. at a temperature of 20 ~25°C and a relative humidity 40%~70%, and bright and dark illumination for 12 hours respectively; these mice were allowed to drink and eat freely. After normal feeding for about 6 days, mice with good physical signs can be selected for this experiment after veterinary inspection. Before grouping, animals were identified at their base of tail using marking pens. After grouping, each animal was identified by ear clipping.

Human prostate cancer cells VCaP were obtained from the Shanghai Cell Bank of the Chinese Academy of Sciences (CAS, cryopreserved in liquid nitrogen in our laboratory).

Under a culture condition of 5% CO2 and 37°C, VCaP cells were routinely cultured in DMEM medium containing 10% fetal bovine serum. Digested with 0.25% trypsin and passaged, VCaP cells were passaged 2-3 times per week based on cell growth, with a passage ratio of 1:3 to 1:5.

he VCaP cells in logarithmic growth phase were collected, counted, and resuspended in 50% DMEM basal medium and 50% Matrigel Matrix, and the cell concentration was adjust to 5 × 107 cells/mL; the cells was placed in an ice box; Cell suspension was aspirated with a 1 mL syringe, and injected subcutaneously to the anterior right armpit of nude mice. Each animal was inoculated with 200μL (1×107 cells/mouse), and a VCaP transplant tumor model in nude mice was established. Regularly observe the status of mice, measure the diameter of the tumor with an electronic vernier caliper, input the data into Excel spreadsheets, calculate the tumor volume, and monitor its growth. When the tumor volume reached 100-300 mm3, 60 mice with good health and similar tumor volume were selected, and divided into 10 groups (n=6) based on the tumor volume using random block method, while ensuring that the average body weight of each group remained consistent as much as possible. The day of grouping was taken as the first day of the experiment (D1). The tumor diameters were measured twice a week after the initiation of the experiment, the tumor volumes were calculated, and the body weights of the animals were weighed and recorded at the same time.

The calculation formula of the tumor volume (TV) was as follows:

$$TV\ (mm3) = 1 \times w2/2$$

wherein, 1 represents the long diameter of the tumor (mm); w represents the short diameter of the tumor (mm).

Preparation of DMSO&PEG400&PG (v:v:v, 10:60:30): appropriate volumes of DMSO, PEG400 and PG were drawed respectively, and mixed well. The ratio of DMSO, PEG400 and PG in the mixture is 10:60:30 (v:v:v), and this mixture is used as a blank solvent and stored at room temperature.

Preparation of the dosing formulations

Appropriate amount of the test compound was weighed and added into a glass bottle, appropriate volume of solvent DMSO was added, be treated with vortex ultrasonication, then appropriate volumes of PEG400 and PG were added in turn, be vortex mixed to obtain a dosing formulation with a final concentration of 0.05M. The dosing formulation was stored in refrigerator at 2-8°C after subpackaging, and was stirred evenly with vortex before each administration. The dosing formulation was prepared every 7 days, and was subpackaged and stored in refrigerator at 4°C.

Preparation of the ZONK1901-10 dosing formulations

Appropriate amount of ZONK1901-10 was weighed and added into a glass bottle, appropriate volume of corn oil, was added, be treated with vortex ultrasonication evenly, a dosing formulation with a final concentration of 19.58 mg mL-1 (0.05 M) was obtained. The dosing formulation was stored in refrigerator at 2-8°C after subpackaging, and was stirred evenly with vortex before each administration. The dosing formulation was prepared every 7 days, and was subpackaged and stored in refrigerator at 4 °C.

The grouping of animals and the dosing regimens were shown in Table 1. The test samples were administered on the day of grouping, and the experiment was ended 28 days later or when the tumor volume of solvent control group reached 2000 mm3 (whichever was reached earlier), and all the administration volume was 10 mL·kg-1. Group A was the blank solvent group, and the blank solvent (DMSO&PEG400&PG(v:v:v, 10:60:30)) was administered intragastrically, once a day (QD). Other groups were intragastrically administered the test compounds, once a day (QD). Group I was intragastrically administered ZONK1901-10 at a dose of 195.8 mg· kg-1 (0.5 mmol kg-1), once a day (QD).

Table 1. Dosing regimen for drug efficacy experiment of nude mouse transplanted tumor model

| **Group** | **Sample** | **Number of animals** | **Dosage (mmol.kg$^{-1}$)** | **Administ ration volume (mL·kg$^{-1}$)** | **Administration route** | **Administration frequency** |
|---|---|---|---|---|---|---|
| ***A | DMSO&PEG400&PG (v:v:v, 10:60:30) | 6 | - | 10 | i.g. | QD |
| **B** | Example 1 | 6 | 0.5 | 10 | i.g. | QD |
| **C** | Example 2 | 6 | 0.5 | 10 | i.g. | QD |
| **D** | Example 3 | 6 | 0.5 | 10 | i.g. | QD |
| **E** | Example 4 | 6 | 0.5 | 10 | i.g. | QD |
| **F** | Example 5 | 6 | 0.5 | 10 | i.g. | QD |
| **G** | Example 6 | 6 | 0.5 | 10 | i.g. | QD |
| **H** | Example 7 | 6 | 0.5 | 10 | i.g. | QD |
| **I** | ZONK1901-10 | 6 | 0.5 | 10 | i.g. | QD |
| **J** | ZONK1901-19 | 6 | 0.5 | 10 | i.g. | QD |

On the last day of the experiment, the animals were euthanized (CO2) after weighing the body weight and measuring the tumor diameter. The tumor tissue was stripped, weighed and photographed, and the tumor weight inhibition rate was calculated. The animals were dissected grossly, and the internal organs were visually observed for abnormalities. Animal carcasses and tumor tissues were stored in refrigerator for unified treatment.

Data record, calculation formula

The calculation formula of relative tumor volume (RTV) is:

$$RTV = TVt/TVinitial$$

wherein, TVinitial is the tumor volume measured during group administration; TVt is the tumor volume at each measurement during administration.

The calculation formula of relative tumor proliferation rate (%T/C) is:

$$\%T/C = 100\% \times (RTVT/RTVC)$$

wherein, RTVT represents the RTV of the treatment group;; RTVC epresents the RTV of the solvent control group.

The calculation formula of tumor growth inhibition rate TGI (%) is:

$$TGI = 100\% \times [1-(TVt(T)-TVinitial(T))/(TVt(C)-TVinitial(C))]$$

wherein, TVt(T) represents the tumor volume measured each time in the treatment group; TVinitial(T) represents the tumor volume of the treatment group measured during group administration; TVt(C) represents the tumor volume measured each time in the solvent control group; TVinitial(C) represents the tumor volume of the solvent control group during group administration.

The calculation formula of animal weight loss rate is:

$$\text{Animal weight loss rate} = 100\% \times (\text{BWinitial} - \text{BWt})/\text{BWinitial}$$

Wherein, BWt represents the animal body weight measured each time during the administration period; BWinitial represents the animal body weight at the time of group administration.

The calculation formula of tumor weight inhibition rate IR (%) is:

$$\text{IR} = 100\% \times (\text{WC} - \text{WT})/\text{WC}$$

Wherein, WC represents the tumor weight in the solvent control group; WT represents the tumor weight in the treatment group.

Tumor volume and tumor growth inhibition rate of animals in each group during the administration period

| Group | A (solvent) | B | C | D | E | F | G | H | I | J |
|---|---|---|---|---|---|---|---|---|---|---|
| Day 1 | 226±12 | 228±14 | 230±12 | 225±7 | 238±16 | 280±15 | 227±21 | 228±14 | 274±18 | 252±21 |
| day 25 | 1103±66 | 384±32 ** | 393±25 ** | 478±64 ** | 377±37 ** | 410±44 ** | 405±15 * | 492±30 ** | 765±46 * | 790±49 * |
| Growth Inhibition rate (GI) | | 82.2% ** | 81.4% ** | 71.2% ** | 84.2% ** | 85.2% ** | 80.7% ** | 69.3% ** | 44.1%* | 38.7%* |
| Note: "*" indicates that there is a significant difference between the tumor volume and the solvent control group (P<0.05); "**" indicates that there is a very significant difference between the tumor volume and the solvent control group (P<0.01) | | | | | | | | | | |

Example 20: pharmacokinetic experiment

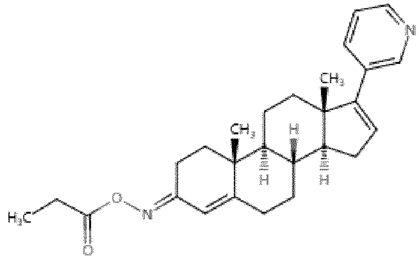

ZONK1901-19(Group H)

To study the pharmacokinetic properties after intragastric administration in mice.

45 male mice are randomly divided into 9 groups (each group contains 5), embodiment 1 compound (B), embodiment 2 compound (C), embodiment 3 compound (D), embodiment 4 (E), embodiment 5(F), embodiment 6(G), and ZONK1901-19(H), ZONK1901-10(1) (abiraterone acetate).

Each mouse was collected at 2-3 discrete points in time.

An LC-MS/MS analysis method for determining the concentrations of ZONK1901-1 and ZONK1901-2 in mouse whole blood was established. At the same time, the obtained plasma concentration data was calculated by using the pharmacokinetic processing software Pharsight Phoenix WinNonlin8.0 non-compartmental model to calculate relevant pharmacokinetic parameters.

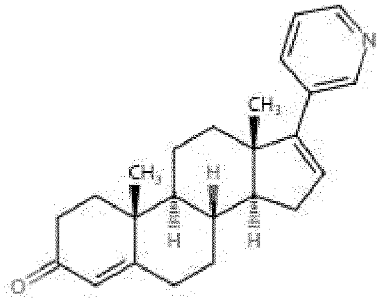

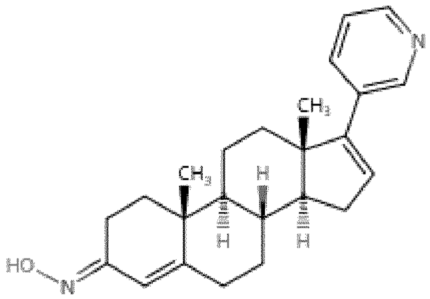

ZONK1901-1 ZONK1901-2

（M1） （M2）

The main pharmacokinetic parameters of the metabolites ZONK1901-1 and ZONK1901-2 after intragastric administration in each group are as follows:

| Gro up | Compoud | $t_{1/2}$ (h) | $T_{max}$ (h) | $C_{max}$ (nM) | $AUC_{0-t}$ (nM·h) | $AUC_{0-inf}$ (nM·h) |
|---|---|---|---|---|---|---|
| B | ZONK1901-1 | 4.58 | 2.00 | 654 | 2156 | 2236 |
| | ZONK1901-2 | 2.92 | 2.00 | 1039 | 3425 | 3702 |
| C | ZONK1901-1 | 2.30 | 2.00 | 1094 | 3916 | 4021 |
| | ZONK1901-2 | 2.75 | 2.00 | 532 | 3125 | 3150 |
| D | ZONK1901-1 | 2.30 | 2.00 | 1258 | 4916 | 4021 |
| | ZONK1901-2 | 2.75 | 2.00 | 568 | 2879 | 3021 |
| E | ZONK1901-1 | 1.35 | 4.00 | 1219 | 6527 | 6554 |
| | ZONK1901-2 | 1.95 | 4.00 | 644 | 4558 | 4669 |

(continued)

| Gro up | Compoud | $t_{1/2}$ (h) | $T_{max}$ (h) | $C_{max}$ (nM) | $AUC_{0-t}$ (nM·h) | $AUC_{0-inf}$ (nM·h) |
|---|---|---|---|---|---|---|
| F | ZONK1901-1 | 2.73 | 2.00 | 661 | 2227 | 2308 |
| | ZONK1901-2 | 3.17 | 2.00 | 398 | 2127 | 2276 |
| G | ZONK1901-1 | 2.73 | 2.00 | 679 | 2103 | 2396 |
| | ZONK1901-2 | 3.17 | 2.00 | 421 | 2237 | 2287 |
| H | ZONK1901-1 | 4.58 | 0.500 | 112 | 554 | 563 |
| | ZONK1901-2 | 2.92 | 0.500 | 103 | 342 | 370 |
| I | ZONK1901-1 | 4.58 | 0.500 | 101 | 454 | 360 |
| | ZONK1901-2 | | NR | NR | NR | NR |

Example 21: Pharmacodynamics evaluation of acne

There are 140 male healthy Japanese big-ear white rabbits, weighing 2.0-2.5kg. After one week of adaptive rearing, 10 rabbits were randomly selected according to their body weights as the normal control group, and the remaining 130 rabbits were coated with coal tar once a day in the area of 2×2cm2 at 2cm outside the opening of ear canal, with a thickness of about 0.5mm (Before coating, the coal tar was heated to melt in a water bath, and the last coal tar shell was removed before each application; the rabbits in normal control group were coated with olive oil by the same method as before). After 3 weeks for modeling, according to the degree of skin lesion, 130 rabbits were randomly divided into 13 groups which is model control group, Example 1 compound (1 group), Example 2 compound (2 groups), Example 3 compound (3 groups), Example 4 ( 4 groups), Example 5 (5 groups), Example 6 (6 groups), Example 7 (7 groups), Example 8 (8 groups), Example 9 (9 groups), Example 10 (10 groups ), Example 11 (11 groups), Example 12 (12 groups), Example 13 (13 groups), and each group had 10 rabbits. After grouping, each dose group were continued to be coated coal tar in the morning, and after the coal tar was removed in the afternoon, apply the test drug or control drug (both 0.01%/10g ointment, apply 1g ointment per animal) on the lesion, once a day, for two consecutive weeks (the model control group and the normal control group were coated with the same amount of matrix). 24 hours after the last administration, the animals were sacrificed by air embolism, and the skin (full thickness) of the ear where the administration was administered was taken, fixed with 4% paraformaldehyde solution, stained with HE, and routinely observed for pathology.1.

1 , Experimental results

Effects on keratinization of hair follicles in experimental rabbit ear acne

| Group | Degree of keratinization | | | | |
|---|---|---|---|---|---|
| | - | + | ++ | +++ | |
| normal control group | 10 | 0 | 0 | 0 | ** |
| model control group | 0 | 0 | 5 | 5 | |
| 1 | 0 | 6 | 4 | 0 | * |
| 2 | 0 | 5 | 5 | 0 | * |
| 3 | 0 | 6 | 4 | 0 | * |
| 4 | 1 | 7 | 3 | 0 | * |
| 5 | 0 | 6 | 4 | 0 | * |
| 6 | 0 | 5 | 5 | 0 | * |
| 7 | 0 | 6 | 4 | 0 | * |
| 8 | 0 | 5 | 5 | 0 | * |
| 9 | 0 | 6 | 4 | 0 | * |
| 10 | 1 | 6 | 3 | 0 | * |

(continued)

| Group | Degree of keratinization | | | | |
|---|---|---|---|---|---|
| | - | + | ++ | +++ | |
| 11 | 0 | 7 | 3 | 0 | * |
| 12 | 0 | 5 | 5 | 0 | * |
| 13 | 0 | 4 | 6 | 0 | * |
| *means P<0.05, compared with the model control group; **means P<0.01, compared with the model control group; | | | | | |

It can be seen from the above table that compared with the model control group, the keratinization in the hair follicles of the rabbit ears in each group was improved.3.2 Effects on the degree of inflammatory cell infiltration in the experimental rabbit ear acne model

| Group | degree of inflammatory cell infiltration | | | | |
|---|---|---|---|---|---|
| | - | + | ++ | +++ | |
| normal control group | 10 | 0 | 0 | 0 | ** |
| model control group | 0 | 0 | 5 | 5 | |
| 1 | 0 | 5 | 3 | 2 | * |
| 2 | 0 | 4 | 4 | 2 | * |
| 3 | 0 | 5 | 4 | 1 | * |
| 4 | 0 | 5 | 3 | 2 | * |
| 5 | 0 | 6 | 3 | 1 | * |
| 6 | 0 | 6 | 3 | 1 | * |
| 7 | 0 | 5 | 3 | 2 | * |
| 8 | 0 | 4 | 4 | 2 | * |
| 9 | 0 | 5 | 4 | 1 | * |
| 10 | 0 | 5 | 3 | 2 | * |
| 11 | 0 | 6 | 3 | 1 | * |
| 12 | 0 | 5 | 4 | 1 | * |
| 13 | 0 | 6 | 3 | 1 | * |
| ** indicates that compared with the model control group, P<0.01; * indicates that compared with the model control group, P<0.05 | | | | | |

It can be seen from the above table that compared with the model control group, the infiltration of otitis cells in rabbits in each group has improved.

## Claims

**1.** , A compound of formula I:

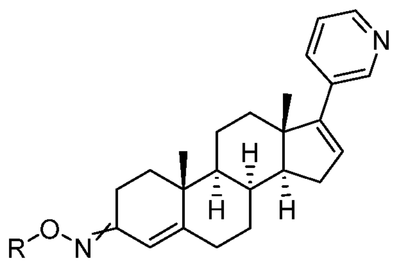

formula I,

In formula I, R is selected from:

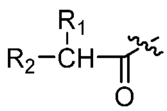 , 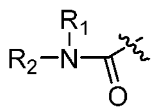 , 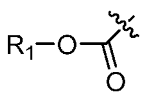 , 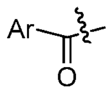

, Ar represents a substituted or unsubstituted aromatic ring, specifically a benzene ring or a pyridine ring; the substituent could be C1-C8 alkyl group, C1-C8 alkoxy group, or halogen;
wherein, R1 or R2 is independently selected from any one of the following groups: H, (C3-C8) carbocycloalkyl, substituted or unsubstituted (C1-C18) alkane, (C2-C8) alkenes or (C2-C8) alkenyl containing substituents, (C2-C8) alkynyl or (C2-C8) alkynyl containing substituents, (C6-C20) aryl or (C6-C20) aryl containing substituents, (C2-C20) heterocyclic or (C2-C20) heterocyclic containing substituents;
alternatively, R1 and R2 form a ring with each other as (C3-C8) heterocycloalkyl or substituted (C3-C8) heterocycloalkyl, (C6-C20) heteroaryl or substituted (C6-C20) heteroaryl;
when R is:

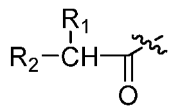

, R1 or R2 is not alkane with 1 or more unsubstituted carbon atoms.

**2.** . Abiraterone derivative shown in formula I of claim 1,wherein the abiraterone derivative has any one of the following structure:

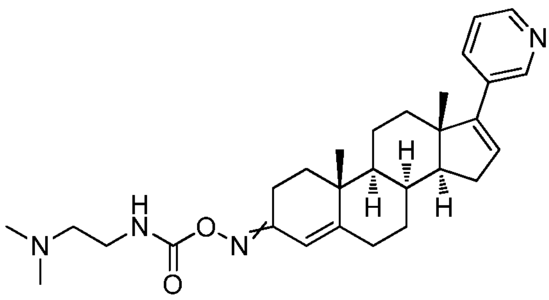

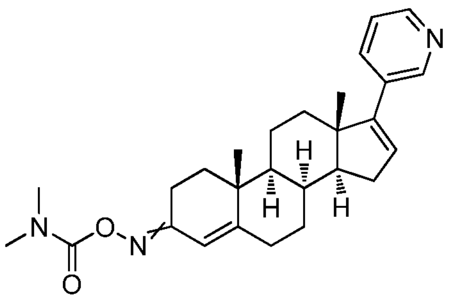

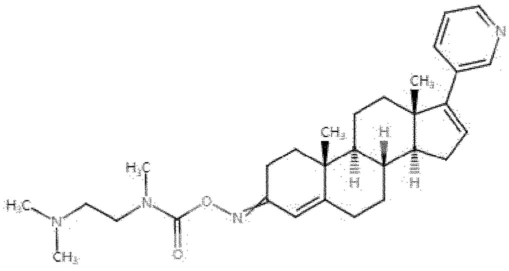

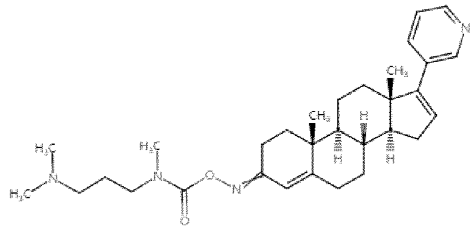

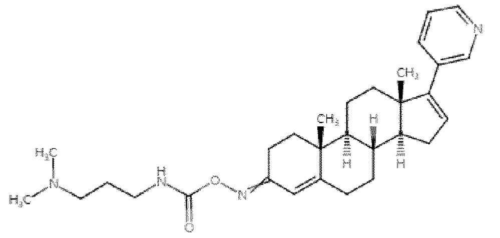

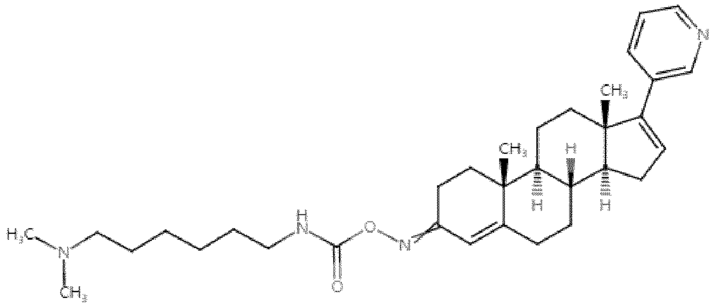

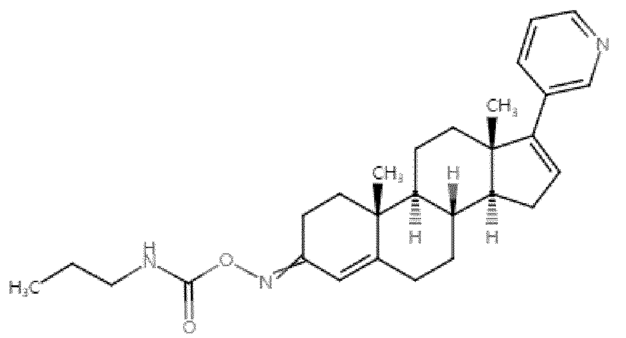

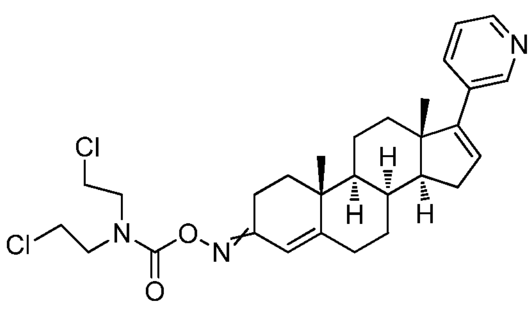

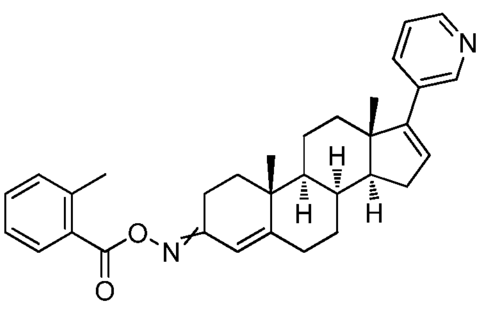

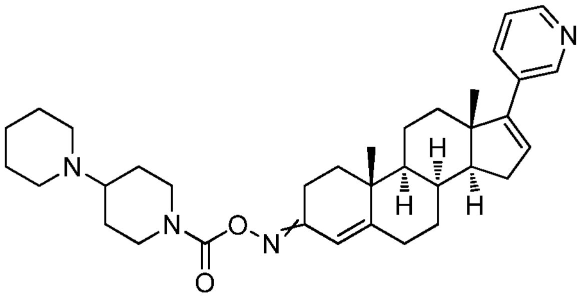

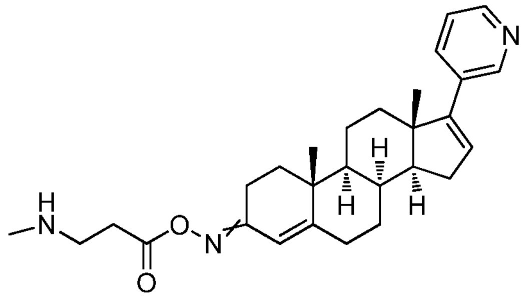

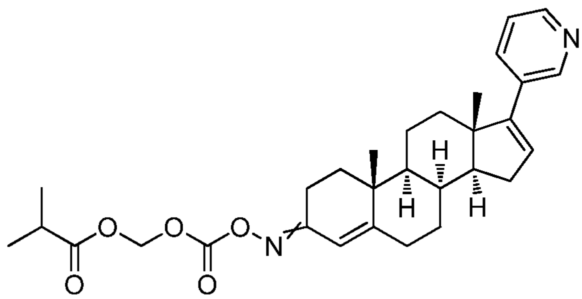

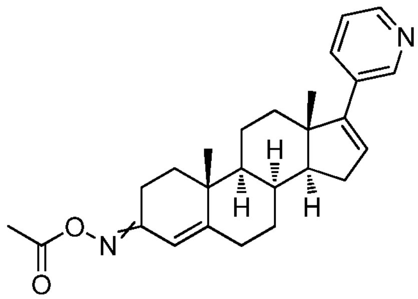

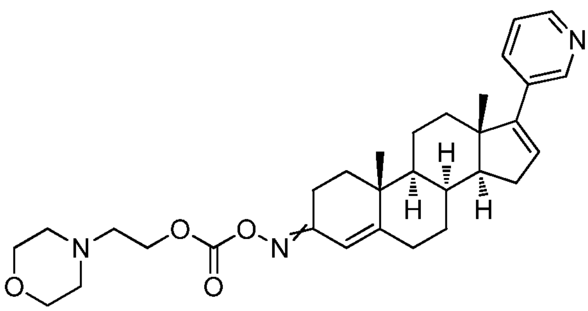

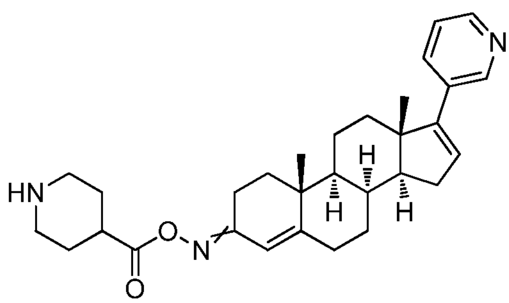

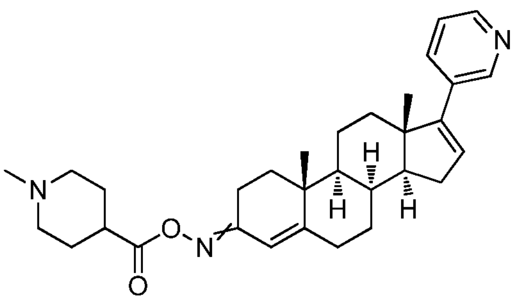

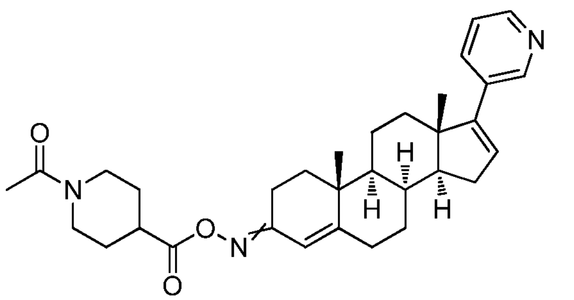

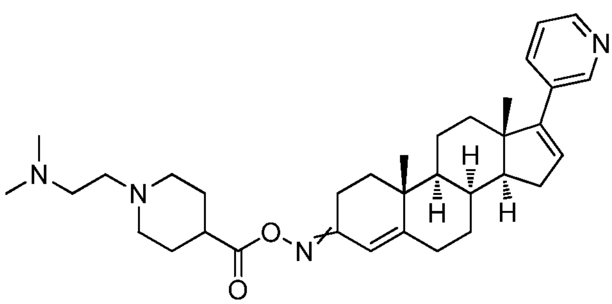

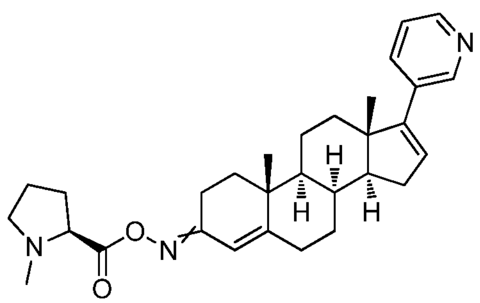

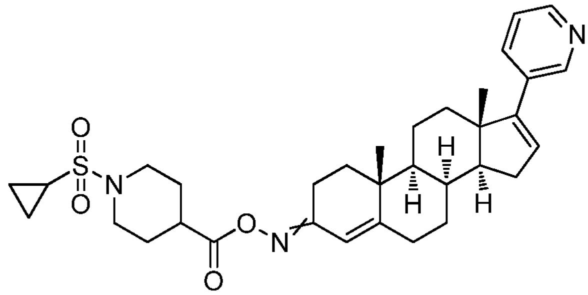

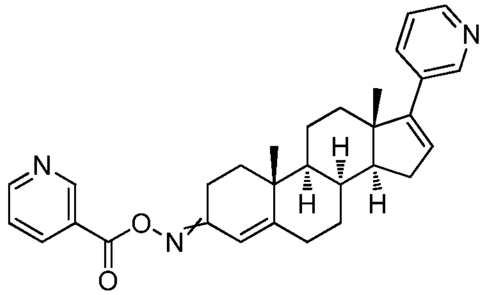

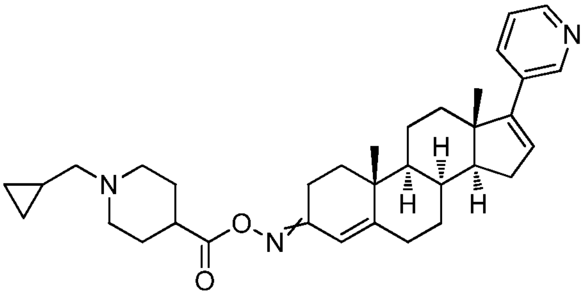

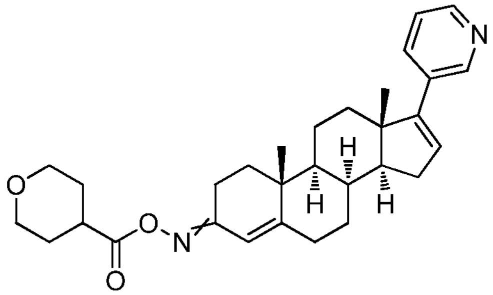

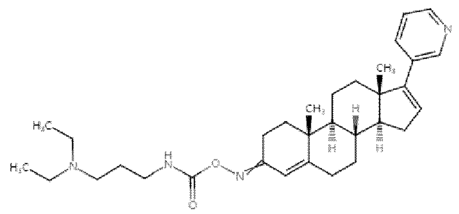

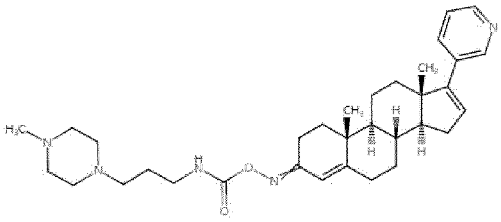

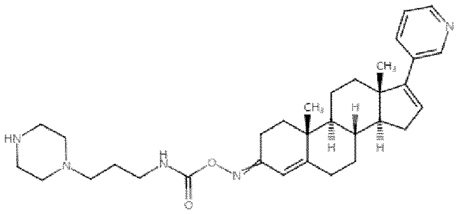

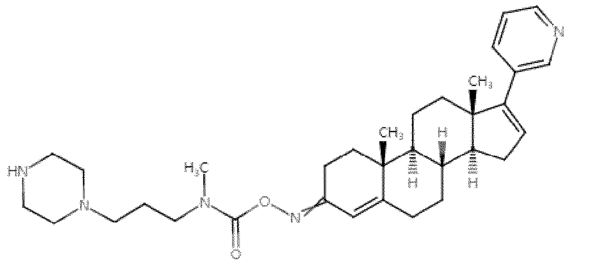

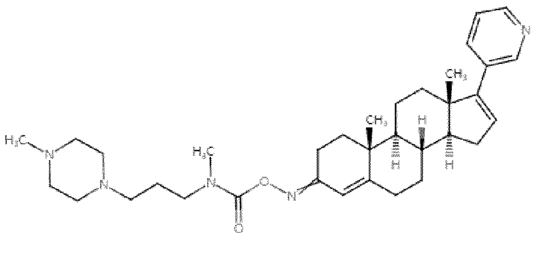

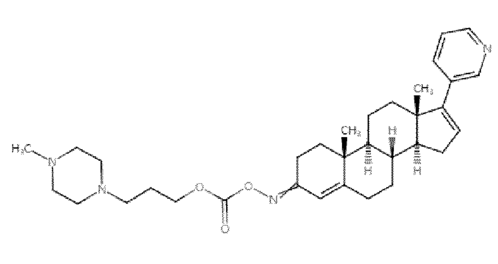

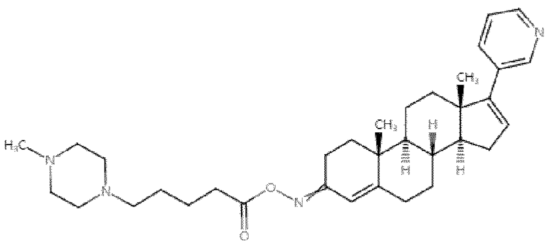

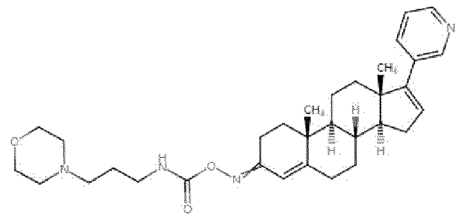

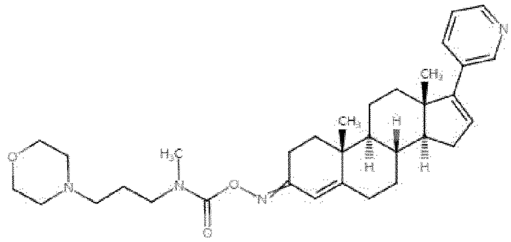

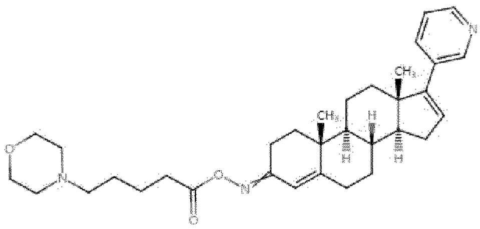

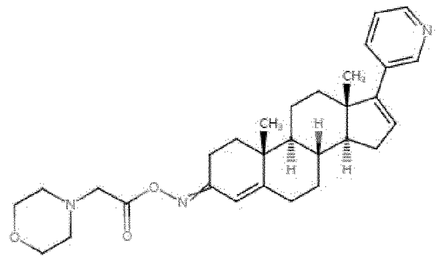

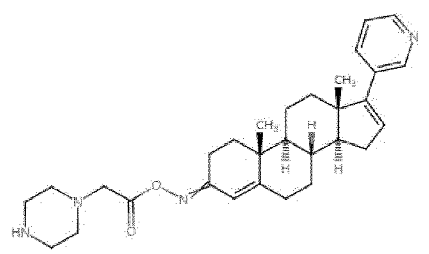

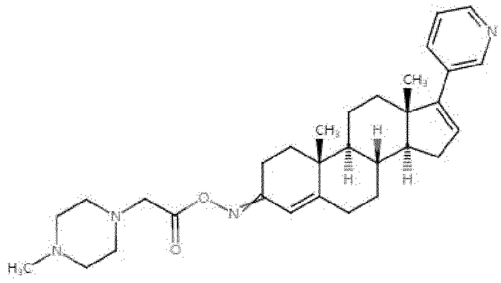

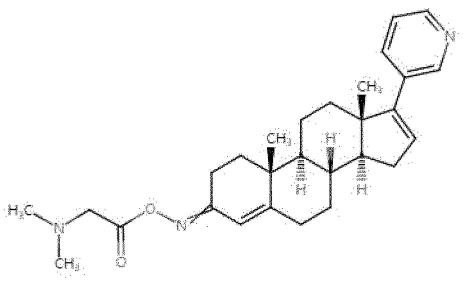

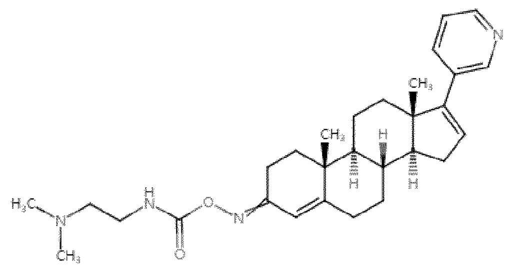

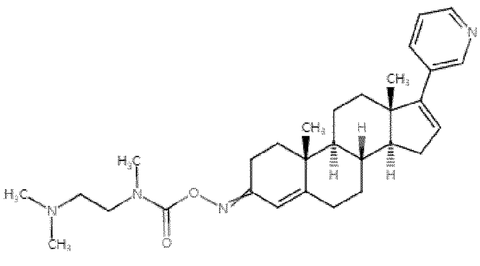

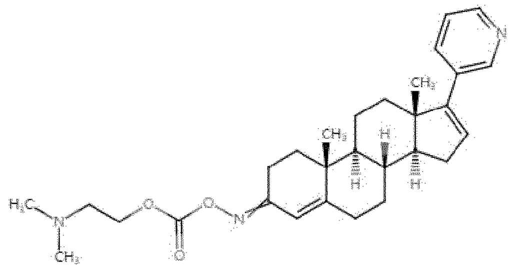

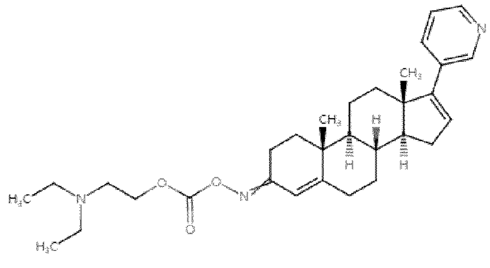

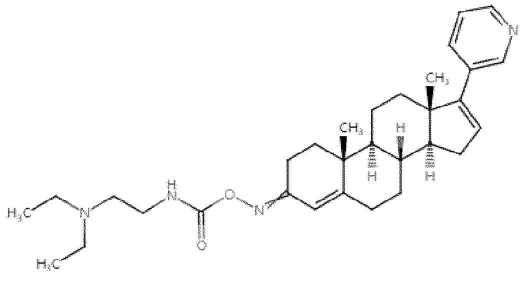

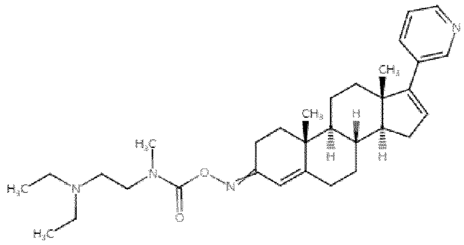

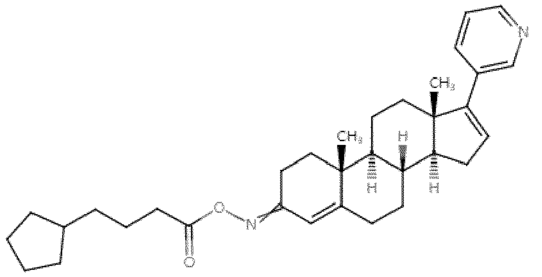

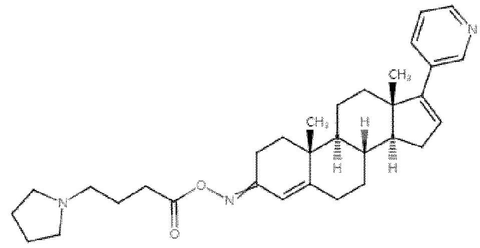

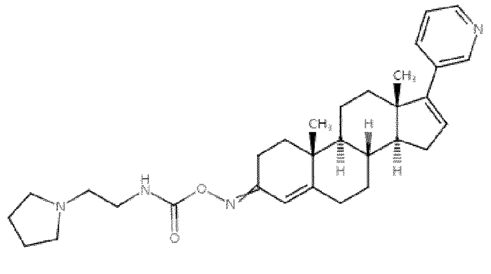

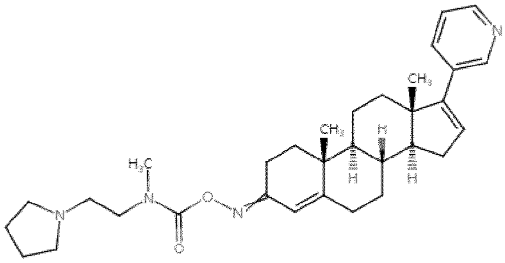

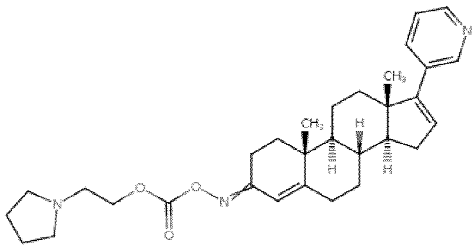

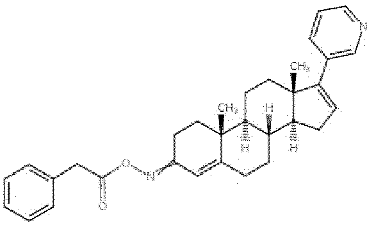

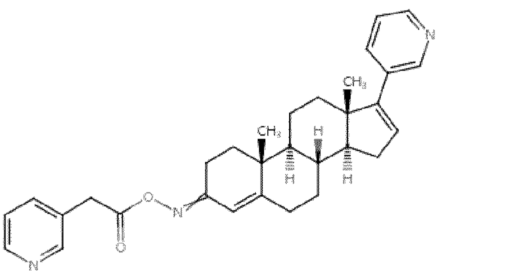

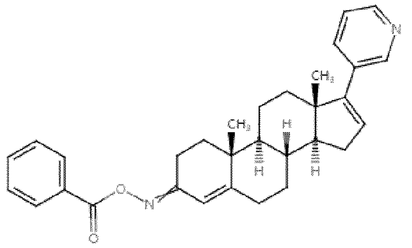

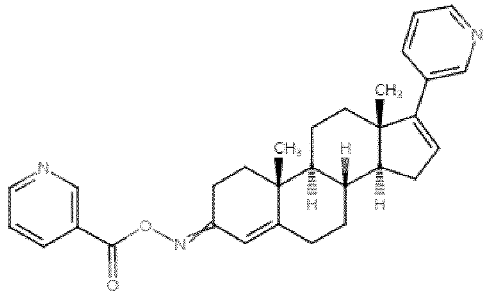

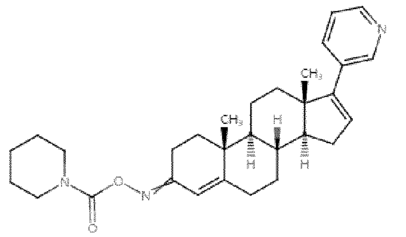

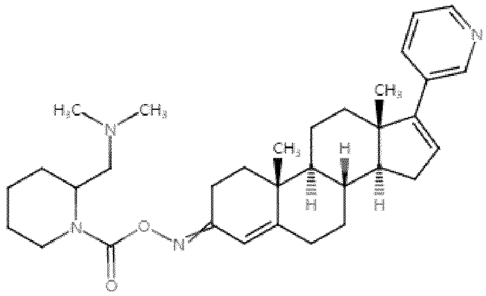

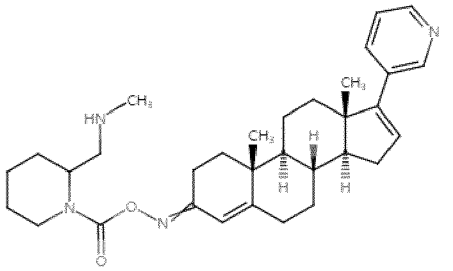

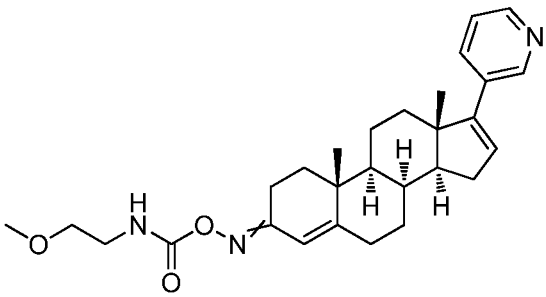

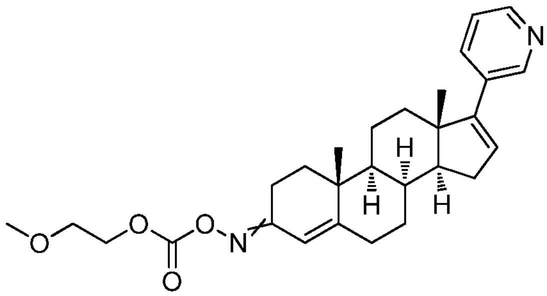

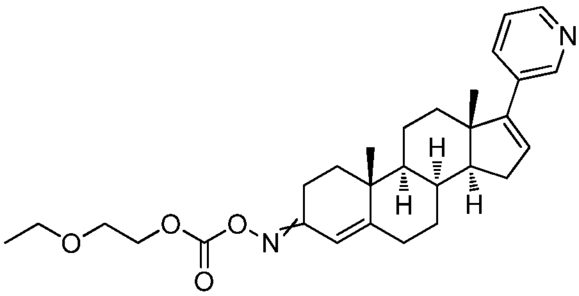

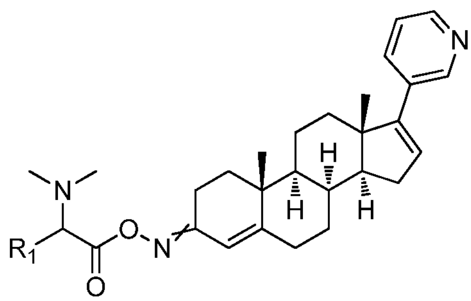

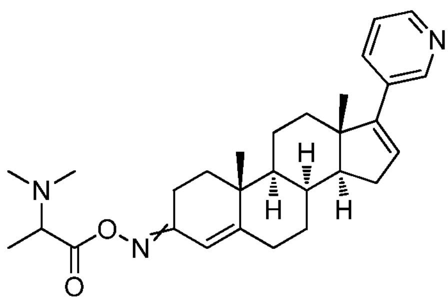

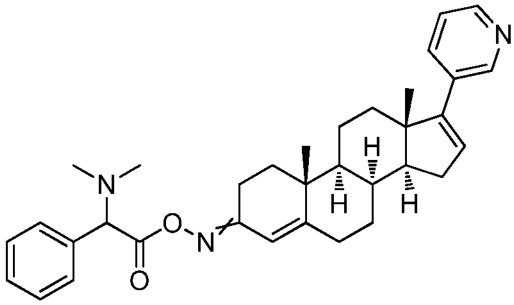

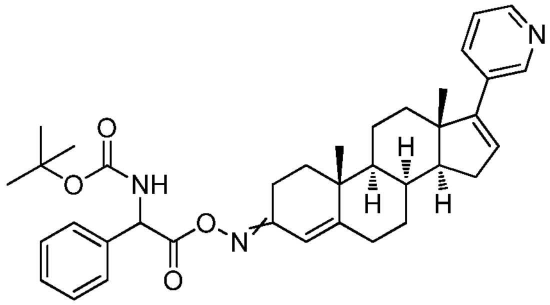

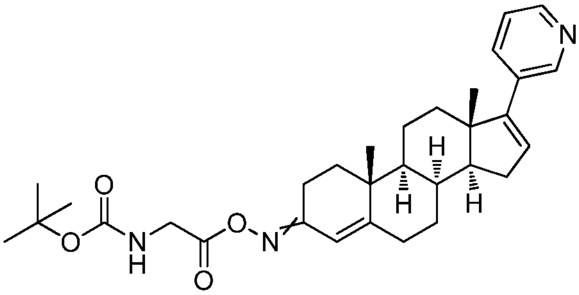

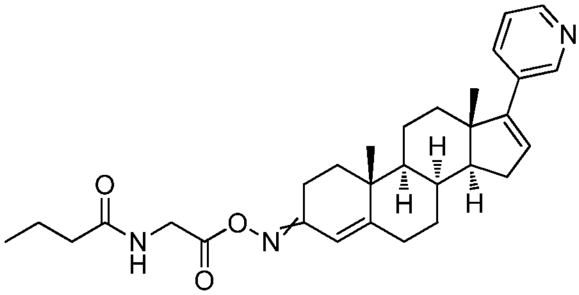

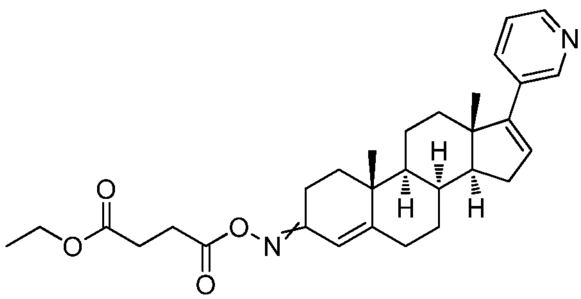

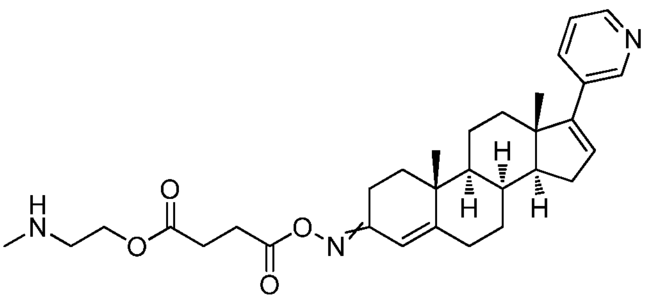

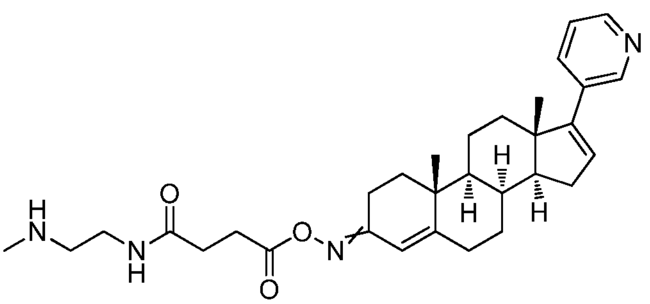

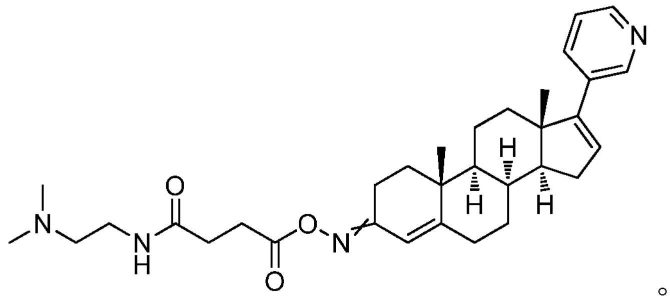

3. . Use of abiraterone derivative shown in formula I according to claim 1 or 2,

1 ) for the preparation of eukaryotic tumor cell proliferation inhibitors; or
2) for the preparation of a drug for the prevention and/or treatment of tumors.

4. .The use of abiraterone derivative according to claim 3, wherein,the eukaryote is a mammal;

the tumor cells are cancer cells;
the cancer cells are prostate cancer cells;
the tumor is carcinoma; the carcinoma is prostate cancer.

5. .The use of abiraterone derivative according to claim 4, wherein, the disease is metastatic castration-resistant prostate cancer after prior docetaxel chemotherapy.

6. .A eukaryotic tumor cell proliferation inhibitor comprises the abiraterone derivative shown in formula I according to claim 1 or 2.

7. .The use of abiraterone derivative according to in claim 1 or 2 for the preparation of a drug for treating acne.

8. .A drug for treating acne, which comprises the abiraterone derivative shown in formula I according to claim 1 or 2.

## INTERNATIONAL SEARCH REPORT

International application No.
**PCT/CN2022/103497**

**A. CLASSIFICATION OF SUBJECT MATTER**

C07J 43/00(2006.01)i; A61K 31/58(2006.01)i; A61P 17/10(2006.01)i; A61P 35/00(2006.01)i; A61P 13/08(2006.01)i

According to International Patent Classification (IPC) or to both national classification and IPC

**B. FIELDS SEARCHED**

Minimum documentation searched (classification system followed by classification symbols)

C07J; A61K; A61P

Documentation searched other than minimum documentation to the extent that such documents are included in the fields searched

Electronic data base consulted during the international search (name of data base and, where practicable, search terms used)

CNTXT; CNKI; VEN; WOTXT; USTXT; EPTXT; STN; ISI Web of Science; 万方, WANFANG: 广东中科药物研究有限公司, 王伟, 陆永章, 谭进辉, 阿比特龙, 衍生物, 痤疮, 癌, 肿瘤, abiraterone, derivative?, acne, cancer, tumor, structural formula (I)

**C. DOCUMENTS CONSIDERED TO BE RELEVANT**

| Category* | Citation of document, with indication, where appropriate, of the relevant passages | Relevant to claim No. |
|---|---|---|
| PX | CN 114106077 A (GUANGDONG ZONK DRUG R & D LIMITED) 01 March 2022 (2022-03-01)<br>claims 1-8 | 1-8 |
| X | CN 106279343 A (GUO MINGSHAN) 04 January 2017 (2017-01-04)<br>claims 1-16 | 1-8 |
| X | CN 107226834 A (LI CHENGFENG) 03 October 2017 (2017-10-03)<br>claims 1-10, and description, paragraph [0018] | 1-8 |
| A | CN 104974212 A (BEIJING LANG REBONE TECHNOLOGY CO., LTD.) 14 October 2015 (2015-10-14)<br>entire document | 1-8 |
| A | CN 110272465 A (CHENGDU BEINUO KECHENG BIOLOGICAL TECHNOLOGY CO., LTD.) 24 September 2019 (2019-09-24)<br>entire document | 1-8 |
| A | CN 106977577 A (HUNAN NORMAL UNIVERSITY) 25 July 2017 (2017-07-25)<br>entire document | 1-8 |
| A | WO 2014111815 A2 (CORTENDO AB PUBL) 24 July 2014 (2014-07-24)<br>entire document | 1-8 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2022** | **28 September 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/103497**

| Patent document cited in search report | Publication date (day/month/year) | Patent family member(s) | Publication date (day/month/year) |
|---|---|---|---|
| CN 114106077 A | 01 March 2022 | None | |
| CN 106279343 A | 04 January 2017 | None | |
| CN 107226834 A | 03 October 2017 | None | |
| CN 104974212 A | 14 October 2015 | None | |
| CN 110272465 A | 24 September 2019 | None | |
| CN 106977577 A | 25 July 2017 | None | |
| WO 2014111815 A2 | 24 July 2014 | CA 2898573 A1 | 24 July 2014 |
| | | WO 2014111815 A3 | 06 November 2014 |
| | | EP 2945960 A2 | 25 November 2015 |
| | | US 2015337003 A1 | 26 November 2015 |

Form PCT/ISA/210 (patent family annex) (January 2015)